# EUROPEAN PATENT SPECIFICATION

(11) **EP 3 338 837 B1**
(45) Date of publication and mention of the grant of the patent: **27.01.2021**
(21) Application number: 15901698.9
(22) Date of filing: 19.08.2015
(51) Int. Cl.: A61M 5/31, A61M 5/178, G09F 3/00

(54) **SYRINGE FOR TYMPANIC INJECTION**
SPRITZE FÜR TROMMELFELLINJEKTION
SERINGUE POUR INJECTION AU TYMPAN

(43) Date of publication of application: 27.06.2018
(73) Proprietor: Sato Pharmaceutical Co., Ltd., Tokyo 107-0051 (JP)
(72) Inventor: SATO, Seiichi, Tokyo 107-0051 (JP); KOYAMA, Yoshitoshi, Tokyo 107-0051 (JP)
(74) Representative: Oxley, Robin John George
(86) International application number: PCT/JP2015/073189
(87) International publication number: WO 2017/029716

(56) References cited:
- EP-A2- 2 525 844
- WO-A1-2013/141187
- WO-A1-2013/141187
- JP-A- 2006 136 417
- JP-A- 2006 136 417
- JP-A- 2008 541 945
- JP-A- 2009 050 289
- JP-A- 2013 517 841
- JP-A- 2015 517 368
- US-A1- 2015 051 579

## Description

### Technical Field

The present invention relates to a syringe for tympanic injection for directly injecting an infusion in a barrel into a tympanic cavity.

### Background Art

The intratympanic administration usually refers to a technique for injecting a liquid medicine with a needle through the tympanic membrane into the tympanic cavity. The liquid medicine injected into the tympanic cavity further spreads via the round window membrane to the inner ear (it is anatomically difficult to directly inject a liquid medicine to the round window membrane located inside of the tympanic membrane).

In recent years, attempts to administer infusions into the tympanic cavity have been made for the treatment of a plurality of diseases and the palliation of symptoms. For example, a change over time in potassium ion concentration in the scala tympani after intratympanic injection of potassium chloride solutions has been examined in studies to investigate the cause of attacks of Meniere's disease and the treatment of tinnitus by intratympanic injection of Xylocaine and steroids has been studied as a therapy for tinnitus. Moreover, a therapy for acute suppurative otitis media and chronic suppurative middle ear disease with acute exacerbation by intratympanic injection of Neuzym for local administration has been studied as a therapy for otitis media. Furthermore, intratympanic injection of steroids has been reported as a study of therapies for idiopathic sudden hearing loss.

For example, as to idiopathic sudden hearing loss, many still remain to be revealed on the cause of the onset and systemic steroid therapies, hyperbaric oxygen therapies, and the like have been attempted conventionally as common therapies.

The systemic steroid therapies have had a problem that only the small portion of the administered steroid achieves the inner ear and the cure rate is not high as well. In such a circumstance, the efficacy of intratympanic steroid therapies alone or in combination with a conventional therapy as therapies for sudden hearing loss has been examined in recent years. The intratympanic steroid therapies are therapies in which a small hole through the tympanic membrane is made and a steroid is directly injected into the tympanic cavity.

Non-Patent Literature 1 and Non-Patent Literature 2 report that while the cure rate (including cure, marked recovery, recovery) by a combination of 2 therapies of systemic steroid and hyperbaric oxygen therapies is 22.6%, the cure rate (including cure, marked recovery, recovery) by a combination of 3 therapies of systemic steroid and hyperbaric oxygen therapies plus an intratympanic steroid therapy with as high as 75.8%.

Meanwhile, adverse events in the intratympanic steroid therapy have been reported to include 44% dizziness and 24% nausea (however, all adverse events disappeared during the observation period, indicating that the therapy is a safe therapy).

Symptoms such as dizziness and nausea at the time of such injection of the liquid medicine into the tympanic cavity are supposed to be caused by the difference of temperatures of the liquid medicine and the body. The tympanum is a part very sensitive to stimulations from the outside, e.g., temperature difference. Therefore, a liquid medicine is currently administered after reducing the temperature difference between the liquid medicine and the body by the doctor's gripping the barrel containing the liquid medicine with hand just before the administration to warm the liquid medicine to near the body temperature empirically or by warming the barrel to near the body temperature with a warming unit such as a heater. However, there has been no way to know whether the warmed liquid medicine maintains the temperature suitable for injection and there has been a possibility of adverse events such as dizziness due to decrease of temperature of the liquid medicine by being exposed to room temperature till injection into the tympanic cavity after the warming.

As a unit for confirming whether the temperature of a liquid medicine is near the temperature suitable for the injection at the time of injection, a syringe that allows visual recognition of temperature abnormality of a liquid medicine in a barrel is disclosed in Patent Literature 1. Specifically, Patent Literature 1 discloses a barrel for liquid medicine an inner circumferential surface of which is covered with a temperature-sensitive elastic sheet body that changes in color with temperature. With the syringe of Patent Literature 1, it can be visually recognized by a change in the color of the temperature-sensitive elastic body that a liquid medicine is out of the range of temperature suitable for the injection and the possibility that the patient feels dizziness or the like because of the injection of such a liquid medicine is avoided.

Patent Literature 2 discloses an injector in which a plurality of heat-sensitive labels are attached to outer circumferential surface of a barrel. According to the disclosure, a method for recording a variety of information irreversibly on heat-sensitive labels on a barrel by a heat writing unit provided in an automatic sucking machine is disclosed. The information is information on the kind of liquid medicine, the amount, the expiration date, the suitable injection speed, or the suitable injection pressure.

### Prior Art Literature

### Patent Literature

[Patent Literature 1] Japanese Patent Laid-Open No. 2005-270579
[Patent Literature 2] Japanese Patent Laid-Open No. 2009-50289

### Non Patent Literature

[Non Patent Literature 1] British Journal of Anaesthesia 96 (2): 259-61 (2006)
[Non Patent Literature 2] Nippon Jibiinkoka Gakkai Kaiho vol.117 (2014), No. 6 p.802-808
   Attention is drawn to the disclosure of US2015/051579.

### Summary of Invention

### Problems to be solved by the Invention

According to the syringe of Patent Literature 1, it is not disclosed that a temperature-sensitive elastic body can define a temperature range at which the liquid medicine should be injected. Therefore, there may be cases where the liquid medicine is warmed to a high temperature above the temperature range at which the liquid medicine should be injected and yet the temperature-sensitive elastic body does not change in color and in such cases the patient may feel dizziness or the like by the heat stimulation of the high temperature liquid medicine.

Furthermore, according to the syringe of Patent Literature 1, the inner circumferential surface of the cylinder member is covered with the temperature-sensitive elastic body. Therefore, the temperature-sensitive elastic body will come in direct contact with the liquid medicine, which makes it necessary to prove the safety that certifies that the component of the temperature-sensitive elastic body does not affect the liquid medicine. Examples of such a proof include complying "Test Methods for Plastic Containers" in General Tests of Japanese Pharmacopoeia.

According to the injector of Patent Literature 2, the information about the liquid medicine in the barrel can be conveniently confirmed by recording various information on the heat-sensitive labels. However, the heat-sensitive labels on the injector according to Patent Literature 2 is used for only the purpose of the record of the information and have a configuration in which the displayed contents inevitably changes irreversibly by heating, and therefore it is not possible to be recognized whether the temperature of the liquid medicine changes over time according to the ambient temperature change is in the predetermined range.

Furthermore, the tympanum is a part sensitive to stimulations such as temperature difference and the dose of the liquid medicine is 1 ml or less and usually around 0.5 ml, which is a very small amount compared to those for other administration sites. Therefore, the outer diameter of the barrel of the syringe to be used is usually as small as 10 mm or less. Because of the small amount of the liquid medicine, even when the syringe filled with the liquid medicine is warmed in an incubator beforehand, the temperature of the liquid medicine starts to be decreased by being placed at room temperature after the removal from the incubator. Therefore, in the intratympanic injection, it has been a very important issue to heat the syringe filled with the liquid medicine to near the body temperature and additionally to confirm that the syringe is at a temperature within a preferred temperature range just before the administration.

The present invention has been made in view of the issue described above and an object of the present invention is to provide a syringe for intratympanic injection, which syringe allows safe and secure recognition that an infusion in a barrel is at a temperature within a temperature range suitable for the administration into the tympanic cavity just before injection.

### Means for Solving the Problems

The invention according to claim 1 for achieving the object described above is a syringe for tympanic injection for directly injecting an infusion in a barrel into a tympanic cavity, comprising a plurality of reversible temperature indicators provided on an outer surface of the barrel, the plurality of reversible temperature indicators each reversibly changing in color at a different temperature, wherein an indication based on difference temperatures at which the reversible temperature indicators change in color allows visual recognition of a range of injection temperature suitable for administration into the tympanic cavity.

With this configuration, an indication that allows the visual recognition of the temperature range of the infusion suitable for the intratympanic administration is made possible by setting the temperatures at which 2 or more different reversible temperature indicators change in color at the upper and lower limits of the range of injection temperature of the infusion suitable for the intratympanic administration. Therefore, by seeing the reversible temperature indicators just before injection, a user of the syringe can certainly recognize that the infusion in a barrel is at a temperature within the temperature range suitable for the intratympanic administration. Moreover, since a reversible temperature indicator is used as a temperature indicator, when the temperature of the infusion is below the temperature range suitable for the intratympanic administration, it is possible to warm the infusion with the barrel again and to repeatedly have indications that allow visible recognition whether the warmed infusion is back to a temperature within the temperature range suitable for the intratympanic administration.

Furthermore, the reversible temperature indicators do not come into a direct contact with the infusion since it is provided on the outer surface of the barrel and the safety is secured, which can be said without proving the safety that certifies that the component of the temperature-sensitive elastic body does not affect the liquid medicine.

In the invention according to claim 1, the reversible temperature indicators comprise at least 3 reversible temperature indicators provided and the at least 3 reversible temperature indicators comprise 2 reversible temperature indicators defining the range of injection temperature suitable for administration into the tympanic cavity and an additional reversible temperature indicator changing in color in the range of injection temperature suitable for administration into the tympanic cavity.

With this configuration, a further additional reversible temperature indicator changes in color in the range of injection temperature suitable for administration into the tympanic cavity and therefore it is possible to recognize whether the infusion is in the higher temperature region or the lower temperature region in the injection temperature range, which regions have a border at the temperature at which the additional reversible temperature indicator changes in color.

In this way, the user of the syringe for tympanic injection can recognize the tendency of the temperature change of the infusion within the injection temperature range suitable for the administration into the tympanic cavity and avoid the situation where the temperature of the infusion becomes out of the temperature range for the injection in a short period of time after the confirmation of the reversible temperature indicators and before the injection of the infusion into a patient because the user is unaware that the infusion is at a temperature in the lower temperature region.

The invention according to claim 3 is the syringe for tympanic injection according to claim 1 or 2, wherein an insulator covering the reversible temperature indicators is provided on an outer surface of the barrel.

With this configuration, the reversible temperature indicators can indicate a temperature close to the temperature of the infusion due to the reduction of the effect of the outside air by the insulator, in spite of the tendency for the reversible temperature indicators provided on the outer surface of the barrel to indicate a temperature lower than the temperature of the real infusion under the influence of the outside air. Therefore, the temperature of the infusion can be recognized more precisely and it becomes possible to recognize more precisely that the infusion in the barrel is at a temperature within the injection temperature range suitable for the administration into the tympanic cavity.

The invention according to claim 4 is the syringe for tympanic injection according to claim 3, wherein the insulator is provided to cover a substantially whole area of the outer surface of the barrel.

With this configuration, the reversible temperature indicators can indicate a temperature close to the temperature of the infusion due to the reduction of the effect of the outside air by the insulator as well as the infusion in the barrel is kept warm by the insulator covering the substantially whole area of the outer surface of the barrel, making it possible to maintain within a preferable temperature range for longer time the temperature of the infusion in the syringe removed from the state where the syringe is warmed.

The invention according to claim 5 is the syringe for tympanic injection according to claim 3 or 4, wherein the insulator has a degree of transparency allowing visual recognition of color of the reversible temperature indicators covered by the insulator.

With this configuration, a change in the color of the reversible temperature indicators can be visually recognized through the insulator and therefore the user can recognize the presence or absence of a change in the color with the reversible temperature indicators with the insulator attached and make an injection into a patient. Thus, it is not necessary to remove the insulator from the outer surface of the barrel before the injection of the infusion into the patient to confirm the presence or absence of a change in the color of the reversible temperature indicators.

The invention according to claim 6 is the syringe for tympanic injection according to any one of claims 3 to 5, wherein a space between the insulator and the outer surface of the barrel is an enclosed space layer.

With this configuration, the barrel is kept warm by the enclosed space layer between the insulator and the outer surface of the barrel and the temperature decrease of the infusion in the barrel is suppressed.

The invention according to claim 7 is the syringe for tympanic injection according to claim 6, wherein a distance between an inner face of the insulator and the outer surface of the barrel is 0.5 mm or more and 2.0 mm or less.

With this configuration, the infusion in the barrel can be kept warm more effectively.

### Effects of Invention

According to the present invention, an indication that allows the visual recognition of the temperature range of the infusion suitable for the intratympanic administration is made possible by setting the temperatures at which 3 different reversible temperature indicators change in color at the upper and lower limits of the range of injection temperature of the infusion suitable for the intratympanic administration. Therefore, by seeing the reversible temperature indicators just before injection, a user of the syringe can certainly recognize that the infusion in the barrel is at a temperature within the temperature range suitable for the intratympanic administration.

### Brief Description of Drawings

[Fig. 1] Fig. 1 is a perspective view illustrating a syringe for tympanic injection according to the first embodiment of the present invention.
[Fig. 2] Fig. 2 is a set of photographs illustrating the mode of a change in the color of the first and second reversible temperature indicators.
[Fig. 3] Fig. 3 is a perspective view illustrating a syringe for tympanic injection according to the second embodiment of the present invention.
[Fig. 4] Fig. 4 is a cross-sectional view taken on line V-V of Fig. 3.
[Fig. 5] Fig. 5 illustrates a modification of the insulator.

### Mode for carrying out the Invention

Embodiments of the present invention are described in detail referring to the drawings in the following. The forms described below do not limit the embodiments of the present invention. The temperature range of an infusion considered to be appropriate varies depending on the agent to be administered, the amount of liquid, the individual difference, and the like. Moreover, the shapes, materials, sizes, arrangement, and the like of the members are not limited.

### (First embodiment)

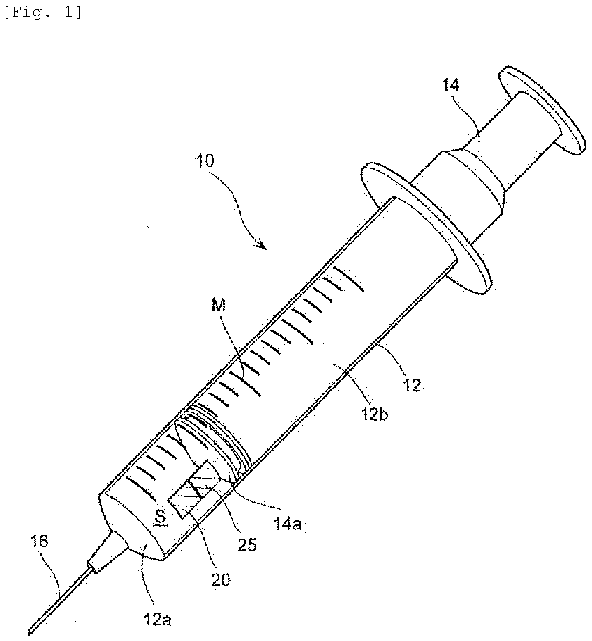

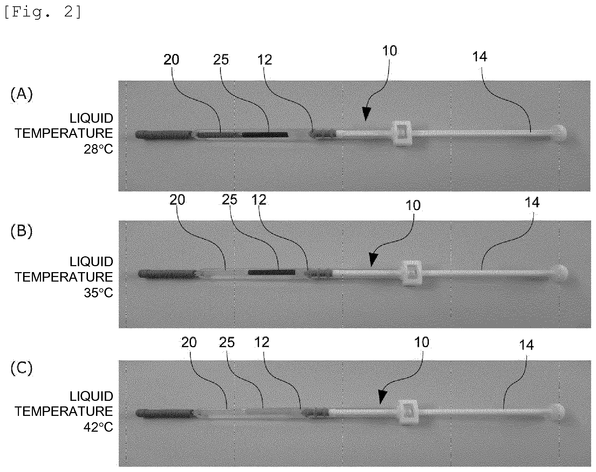

An example of the syringe for tympanic injection according to the first embodiment of the present invention is described with reference to Figs. 1 and 2. Fig. 1 is a perspective view illustrating the syringe for tympanic injection according to the first embodiment of the present invention and Fig. 2 is a set of photographs illustrating the mode of a change in the color of the first and second reversible temperature indicators.

As illustrated in Fig. 1, a syringe for tympanic injection 10 according to the present embodiment is composed of a barrel 12 which is a cylindrical container made of glass or resin and a plunger 14 inserted in the barrel 12 and a space S formed between a piston head 14a, which is a tip of the plunger 14 and a tip 12a of the barrel 12 is filled with an infusion. At the tip 12a of the barrel 12, a needle 16 is formed into the front region thereof. Along the entire central axis of the needle 16, a pore, which is not shown in the figure, is formed and communicates with the space S in the barrel 12.

If the barrel 12 is made of glass, it is required to be made such that alkali does not elute therefrom and the barrel is heat resistant to a high-pressure steam sterilization temperature of 121°C and comply with "Test for Glass containers for Injections" in General Tests of Japanese Pharmacopoeia. If the barrel 12 and the piston head 14a are made of resin, it is required to be made such that they do not adsorb drugs, are heat resistant to a high-pressure steam sterilization temperature of 121°C, and comply with "Test Methods for Plastic Containers" in General Tests of Japanese Pharmacopoeia. Examples of the material of the barrel 12 include polypropylene, polycarbonate, polyethylene terephthalate, polymethyl pentene, cyclic polyolefin, and the like. If the piston head 14a is made of resin, examples of the material of the piston head 14a include a styrene-based elastomer and an elastomer composition comprising an olefin-based elastomer as the main component, for example, a butylene rubber. Furthermore, the piston head 14a made of resin is required to comply with "Test for Rubber Closure for Aqueous Infusions" in General Tests of Japanese Pharmacopoeia.

The plunger 14 is not particularly limited, since it does not come in direct contact with the infusion, and, for example, a plunger made of rigid polyethylene may be used.

The combination of materials of the barrel 12, the piston head 14a, and the plunger 14 is not limited. However, they are necessary to be operated smoothly since carefulness on the pressure applied to the plunger 14 and the amount of the infusion is required in the tympanic injection.

By inserting a part of the needle 16 into a drug container, which is not shown in the figure, that contains an infusion and operating the plunger 14 in the direction to pull the plunger 14 out of the barrel 12, the space S between the tip 12a of the barrel 12 and the tip 14a of the plunger 14 becomes negative pressure and the infusion is aspirated and introduced into the space S through the aforementioned pore of the needle 16. Moreover, use of prefilled syringes for a single use filled with a liquid medicine beforehand is in an increasing tendency in recent years in the view of hygiene and convenience in operation. In this case, S is filled with a required amount of an infusion at the time of production.

The drug may be a compound or a biological formulation and examples thereof include steroids, antimicrobial agents, antibiotics, chemotherapy components, antiviral agents, thrombolytic drugs, vasodepressors, vasoactive materials, antioxidants, painkillers, antiinflammatory agents, local anesthetics, vitamins, and the like. Moreover, 2 or more drugs may be incorporated.

The volume of the infusion in the barrel 12 is an amount suitable for the intratympanic administration and is 1 ml or less, usually about 0.5 ml. Moreover, the diameter of the barrel should be in a range in which the barrel does not disturb the view at the time of the administration of the liquid medicine into the tympanic cavity and is preferably 10 mm or lower.

A scale M is formed on the outer surface 12b of the barrel 12. The user of the syringe 10 recognizes the amount of the liquid medicine in the space S by comparing the positions of this scale M and the tip of the plunger 14 and reading the part of the scale M at which the positions match.

Moreover, on the outer surface 12b of the barrel 12, 2 sheets of the reversible temperature indicators (which are a first reversible temperature indicator 20 and a second reversible temperature indicator 25, respectively) are provided side by side at the positions close to the barrel tip 12a.

The reversible temperature indicators may be those made by encapsulating, for example, cholesteric liquid crystal, a complex pigment such as mercury (II) silver iodide complex, a reversible thermochromic composition, or the like as a heat sensitive ink into microcapsules, kneading the microcapsules into resin, and forming the resin into a sheet-shape, a film-shape, or a tape-shape with one side thereof adhesive. The heat sensitive ink may be directly printed on the outer surface of the barrel.

The reversible temperature indicators may be adjusted in responding temperature range and color at the time of response by selecting materials such as the following as appropriate and blending the materials.

Examples of the complex pigment include iodides of heavy metal such as silver, copper, and lead as well as mercury or complexes thereof.

The reversible thermochromic composition is a composition in which an electron-donating chromogenic compound and an electron accepting compound were dispersed in a solvent compound and the electron-donating chromogenic compound determines the color, the electron accepting compound determines the coloring concentration, and the solvent compound determines the concentration for the color change.

Examples of the electron-donating chromogenic compound include diallylphthalides, polyallylcarbinols, leucoauramines, amylauramines, rhodamine B lactams, indolines, spiropyrans, and fluorans and examples of the electron accepting compound include phenols, carboxylic acids, sulfonic acids, acid phosphoric esters, and metal salts thereof.

Examples of the solvent compound include alcohols, thiols, carboxylic esters, phosphoric ester, sulfonic esters, ketones, ethers, and sulfides.

The sizes of the reversible temperature indicators are not particularly limited, but they are preferably sizes that allow the confirmation of the remained amount of the infusion in the barrel. The shapes thereof are not particularly limited and may each be a number or a character, a square or a circle, or the indicators may be placed around the barrel like a belt such that the color of the temperature indicators can be confirmed from every angle. Moreover, the positions are preferably close to the tip of the barrel such that the temperature is reflected even when the infusion in the barrel is decreased.

The reversible temperature indicators are largely separated into two types in function. The first type develops a color at designated temperature and is colorlessness or a color of the groundwork above or below the temperature and the second type has different colors above and below the designated temperature.

Examples of the former type of the reversible temperature indicators include "Thermo Pit PW" and "Thermo Pit Liquid Crystal Sticker" from AS ONE Corporation and "Thermo-Paint(R)", "Thermowappen(R)", and "Digital thermotape(R)" from NiGK Corporation. For example, the commercially available product "Thermo Pit Liquid Crystal Sticker N-26-46D" has 11 reversible temperature indicators of 26°C, 28°C, 30°C, 32°C, 34°C, 36°C, 38°C, 40°C, 42°C, 44°C, and 46°C printed as numbers on a sticker. "Thermo Pit Liquid Crystal Sticker N-26-46D" exhibits a bright green color at an indicated temperature and a light orange or light blue color at ±1°C of the indicated temperature. For example, at 36°C, only the reversible temperature indicator printed as 36 exhibits a bright green color. At 37°C, the reversible temperature indicator printed as 36 exhibits a light blue color and the reversible temperature indicator printed as 38 exhibits a light orange color.

Specific examples are illustrated in Table 1.

**[Table 1]**

| | | Temperature range of infusion ← suitable for administration into → tympanic cavity | | | | | | |
|---|---|---|---|---|---|---|---|---|
| Temperature (°C) | 33 | 34 | 35 | 36 | 37 | 38 | 39 | 40 |
| First reversible temperature indicator | × | ○ | ○ | ○ | × | × | × | × |
| Second reversible temperature indicator | × | × | × | × | ○ | ○ | ○ | × |

| | | | | | | | | |
|---|---|---|---|---|---|---|---|---|
| ○ Appearance after color change × Appearance before color change | | | | | | | | |

Table 1 is a table illustrating the range of injection temperature suitable for the administration into the tympanic cavity indicated in a way that allows visual recognition by the temperatures at which the first reversible temperature indicator and the second reversible temperature indicator change in color and indicates the case where 34-39°C is the temperature range suitable for the injection. By using "Thermowappen(R)" (special specification: exhibiting a blue color within 35°C±1°C and a black color out of the range) as the first reversible temperature indicator and "Thermowappen(R)" (special specification: exhibiting a green color within 38°C±1°C and a black color out of the range) as the second reversible temperature indicator, the range of injection temperature suitable for administration into the tympanic cavity can be indicated in a way that allows visual recognition

Moreover, by confirming which of the first reversible temperature indicator and the second reversible temperature indicator changes in color in the range of injection temperature suitable for administration into the tympanic cavity, it is possible to recognize whether the infusion is in the higher temperature region or the lower temperature region in the injection temperature range.

Furthermore, the reversible temperature indicators may be not limited as long as they are a plurality of indicators that each change in color reversibly at a different temperature and may be 3, 4, or 5 indicators or the like. Moreover, the reversible temperature indicators may be an assembly made by assembling a plurality of reversible temperature indicators that each change in color reversibly at a different temperature on one sheet. Specifically, it corresponds to "Thermo Pit Liquid Crystal Sticker 30-40" illustrated in [Example 1] described below. This "Thermo Pit Liquid Crystal Sticker 30-40" is an assembly made by assembling on one sheet 6 reversible temperature indicators that change in color reversibly at 30°C, 32°C, 34°C, 36°C, 38°C, 40°C, respectively.

Examples of the latter type of reversible temperature indicators include "Temperature-sensitive dye sticker" from Japan Capsular Products Inc. This is made by preparing a temperature-sensitive dye in microcapsules, kneading the microcapsules into a resin, and forming the resin in a sheet-shape. The temperature of color change and the color are provided upon special orders.

Specific examples are illustrated in Table 2.

**[Table 2]**

| | | Temperature range of infusion ← suitable for administration into → tympanic cavity | | | | | | |
|---|---|---|---|---|---|---|---|---|
| Temperature (°C) | 33 | 34 | 35 | 36 | 37 | 38 | 39 | 40 |
| First reversible temperature indicator 20 | × | ○ | ○ | ○ | ○ | ○ | ○ | ○ |
| Second reversible temperature indicator 25 | × | × | × | × | × | × | × | ○ |

| | | | | | | | | |
|---|---|---|---|---|---|---|---|---|
| ○ Appearance after color change × Appearance before color change | | | | | | | | |

Table 2 is a table illustrating the range of injection temperature suitable for the administration into the tympanic cavity indicated in a way that allows visual recognition by the temperatures at which the first reversible temperature indicator 20 and the second reversible temperature indicator 25 change in color and indicates the case where 34-39°C is the temperature range suitable for the injection. A temperature-sensitive dye sticker (special specification: exhibiting a red color at 33°C or less and being colorless and transparent at 34°C or more) is used as the first reversible temperature indicator 20 and a temperature-sensitive dye sticker (special specification: exhibiting a black color at 39°C or less and being colorless and transparent at 40°C or more) is used as the second reversible temperature indicator 25. By combining the 2 reversible temperature indicators, it can be confirmed whether the temperature is within, above, or below the suitable temperature range.

Next, the mode of a change in the color of the first reversible temperature indicator 20 and the second reversible temperature indicator 25 after warmed the syringe for tympanic injection 10 according to the present embodiment will be described.

First, the barrel 12 is filled with the infusion and the syringe for tympanic injection 10 is warmed to 40°C with a warmer for the exclusive use (not shown in the figure).

When the indicators are transferred from the warmer at room temperature after warming, the first reversible temperature indicator 20 and the second reversible temperature indicator 25 both exhibit the colorless and transparent appearance after color change as illustrate in the raw of 40°C in Table 2, which allows the recognition that the temperature of the infusion exceeds the temperature range suitable for the injection into the human body.

Furthermore, by the transition of the appearance of the second reversible temperature indicator 25 to the appearance before the change into a black color at the time when the liquid temperature reaches 39°C after the infusion is gradually cooled over time under the influence of outside air, it can be known that the temperature of the infusion has reached the temperature range suitable for the injection into the human body (see the raw of 39°C in Table 2).

By the transition of the appearance of the first reversible temperature indicator 20 to the appearance before the change into a red color when the liquid temperature of the infusion is further decreased over time to 33°C, it can be known that the temperature of the infusion becomes below the lower limit of the temperature range suitable for the injection into the human body (see the raw of 33°C in Table 2).

The mode of a change in the color of the first reversible temperature indicator 20 and the second reversible temperature indicator 25 is illustrated in Fig.

2. Fig. 2(A) to (C) illustrates the mode of a change in the color of the first reversible temperature indicator 20 and the second reversible temperature indicator 25 in the cases where the infusion is at 28°C, 35°C, and 42°C, respectively. As illustrated in the figure, the first reversible temperature indicator 20 exhibits a red color at 28°C and the second reversible temperature indicator 25 exhibits a black color. When the temperature of the injection is at 35°C (which is within the temperature range suitable for the administration of the infusion into the tympanic cavity), only the first reversible temperature indicator 20 becomes colorless and transparent. When the temperature of the injection further reaches 42°C, the second reversible temperature indicator 25 becomes colorless and transparent.

Thus, according to the syringe for tympanic injection 10 according to the present embodiment, the range of injection temperature (34 to 39°C) suitable for the administration into the tympanic cavity can be indicated in a way that allows visual recognition by the difference between the temperature at which the first reversible temperature indicator 20 changes in color, which is 34°C, and the temperature at which the second reversible temperature indicator 25 changes in color, which is 40°C.

Table 3 below is a table illustrating the injection temperature range suitable for the administration into the tympanic cavity indicated in a way that allows visual recognition by the temperatures where the first to third reversible temperature indicators change in color.

**[Table 3]**

| | | Temperature range of infusion ← suitable for administration → into tympanic cavity | | | | | | |
|---|---|---|---|---|---|---|---|---|
| Temperature (°C) | 33 | 34 | 35 | 36 | 37 | 38 | 39 | 40 |
| First reversible temperature indicator 20 | × | ○ | ○ | ○ | ○ | ○ | ○ | ○ |
| Second reversible temperature indicator 25 | × | × | × | × | × | × | × | ○ |
| Third reversible temperature indicator 45 | × | × | × | × | ○ | ○ | ○ | ○ |

| | | | | | | | | |
|---|---|---|---|---|---|---|---|---|
| ○ Appearance after color change × Appearance before color change | | | | | | | | |

Table 3 illustrates the case where 34-39°C is the temperature range suitable for the injection. The first reversible temperature indicator 20 is colorless and transparent at 33°C or less and exhibits the appearance after change to a red color at 34°C or more. The second reversible temperature indicator 25 is transparent and colorless at 39°C or less and exhibits the appearance after change to a black color at 40°C or more. The third reversible temperature indicator 45 is transparent and colorless at 36°C or less and exhibits the appearance after change to a blue color at 37°C or more. By combining 3 reversible temperature indicators, it can be confirmed whether the temperature is within, above, or below the suitable temperature range and also whether the temperature is in a higher temperature region or in a lower temperature region in the suitable temperature range.

With any type of indicators, they may be each used as a single temperature indicator or a plurality of the temperature indicators may be placed on one sheet. The ready-made products are commercially available and the temperature for color change, the color, the size, the shape, and the like may be provided according to specific specifications.

The temperature most suitable for the administration of the infusion into the tympanic cavity is the body temperature of the person to be injected and the temperature of the infusion is usually in the temperature range having the center at the body temperature and upper and lower allowances of certain values, preferably in the range of the body temperature ± 3°C, and more preferably in the range of the body temperature ± 2°C.

In this embodiment, there is some temperature difference between the inside of the barrel 12 and the outer surface 12a of the barrel 12. This is because the reversible temperature indicators 20, 25 are affixed to the barrel 12 and the outer surfaces of the reversible temperature indicators 20, 25 in the diameter direction of the barrel are exposed to outside air. Since the temperature of outside air, which is room temperature, is lower than the temperature of the outer surface 12a of the warmed barrel, it is considered that the reversible temperature indicators 20, 25 are affected by the temperatures at both sides.

This temperature difference is affected by the ambient temperature at which the syringe is handled, that is to say, room temperature, the volume of the barrel 12, and the material of the barrel 12. Therefore, when the injection temperature range suitable for the administration into the tympanic cavity is actually defined by using the first reversible temperature indicator 20 and the second reversible temperature indicator 25, the issue can be addressed by shifting the temperatures at which the reversible temperature indicators 20, 25 change in color in consideration of this temperature difference. More specifically, the temperatures at which the reversible temperature indicators change in color will be selected in consideration that there is a temperature difference of 1-2°C depending on the type of the syringe as illustrated in Table 5 below.

As a countermeasure to such cases, one or more other members in addition to a plurality of reversible temperature indicators provided on the outer surface of the barrel may be further provided in this embodiment. It is a unit in which the surfaces of the reversible temperature indicators, which are faces in contact with the air, are covered with an insulator so that the surfaces are not affected directly by the room temperature. Specific examples of the unit include a method involving directly covering the reversible temperature indicators with an insulator and a method involving providing an enclosed space layer between the surfaces of the reversible temperature indicators and the insulator. Further examples include a method involving covering only the surfaces of the reversible temperature indicators and a method involving covering a substantially whole area of the outer surface of the barrel 12b including the surfaces of the reversible temperature indicators. In either case, when an insulator is used, the insulator is desirable to have a degree of transparency at which visual recognition of a change in the color of the reversible temperature indicators through the insulator is possible.

The material of the insulator is not particularly limited, but examples thereof include resins such as high density polyethylene, polypropylene, polyethylene terephthalate, polyester, and the like. These materials are usually colorless and transparent.

Moreover, a closed-cell foam insulation sheet has a numerous number of fine closed air foams in the sheet and high insulation and buffer properties. An insulator made of polyethylene is flexibility and suitable for covering the outer surface of the barrel. Specific examples of such insulators that are commercially available include "Minafoam(R)" (a product made by Sakai Chemical Industry Co., Ltd.), "Lightlon" (a product made by Sekisui Plastics Co., Ltd.), and "Miramat(R)" (a product made by JSP Corporation). The thickness of such a sheet is usually 0.5 mm to 5 mm, but thinner sheets may be obtained according to special specifications. The visual recognition of the color change of a temperature indicator is possible with a thickness of 0.5 mm or less. Insulators with a thickness of 0.3 mm or 0.15 mm have further increased transparency and allow easy visual recognition of a color change. Since the size of the closed-cell foams is 0.1 mm to 0.15 mm, a sheet having a thickness less than 0.15 mm is difficult to produce and inferior in the effect as an insulator.

### (Second embodiment)

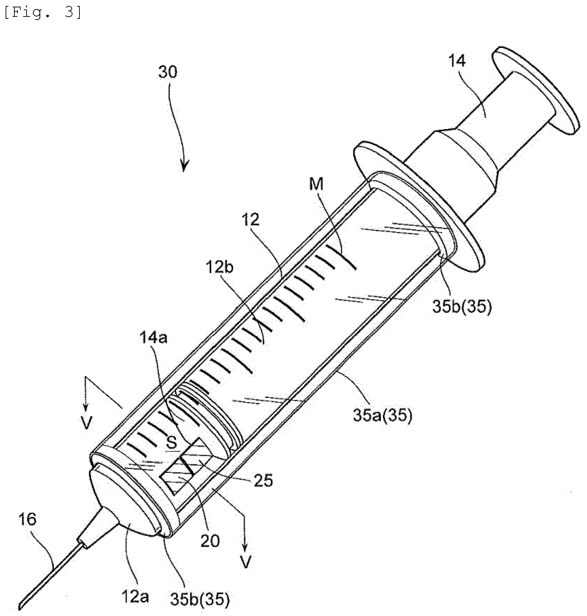

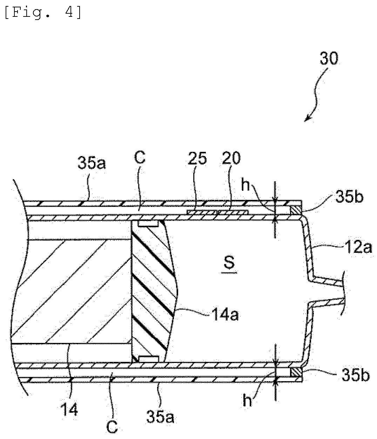

The syringe for tympanic injection 30 in which further members are provided on the outer surface 12b of the barrel 12 will be described below with reference to Figs. 3 and 4. In Figs. 3 and 4, the same elements as those in the embodiment described in Fig. 1 described above are indicated with the same reference signs and the descriptions thereof are omitted. Fig. 3 is a perspective view illustrating the syringe for tympanic injection according to the second embodiment of the present invention and Fig. 4 is a cross-sectional view taken on the line V-V of the figure.

As illustrated in Figs. 3 and 4, the syringe for tympanic injection 30 comprises a sealed space layer C formed by a configuration with a cylinder member 35a extending along the outer surface 12b of the barrel 12 in the longitudinal direction of the barrel 12 and covering the outer surface 12b of the barrel 12 at an outer position in the diameter direction; and a ring-shaped insulator (which is herein referred to as the spacer) 35b locating between the two ends of the cylinder member 35a and the outer surface 12b of the barrel 12 and filling the gap therebetween. The sealed space layer C is filled with air. The thickness h (which is indicated as a distance between the outer surface 12b of the barrel 12 and the inner face of the cylinder member 35a in the present embodiment) of the sealed space layer is about 0.5 mm to 2 mm. When the thickness is 0.5 mm or less, the insulation effect of the sealed space layer is decreased. Moreover, a thickness greater than what is required narrows the field of vision at the time of the administration into the tympanic cavity.

The cylinder member 35a extends with one end located in the direction of the base of the barrel and in contact with a flange 12c and the other end located near the tip 12a of the barrel 12. Thus, the insulator 35 is provided to cover a substantially whole area of the outer surface 12b of the barrel 12.

As illustrated in Table 4, the space between the cylinder member 35a and the outer surface 12b of the barrel 12 is the sealed space layer C enclosed with the spacers 35b, 35b at the both ends in the longitudinal direction. The space between the cylinder member 35a and the outer surface 12b of the barrel 12 in the present embodiment is the sealed space layer C, but it is not necessary to be a completely sealed space layer. More specifically, the enclosed space layer in the present invention may be an enclosed space layer that blocks the aeration between the inside and the outside of the space layer to a certain extent and provides a thermal-insulation effect.

Therefore, the spacers 35b may have a certain degree of permeability that, for example, prevents water from permeating but allows the vapor to pass.

Moreover, the material of the cylinder member 35a preferably has transparency that allows visual recognition of the color change of the reversible temperature indicators 20, 25 covered with the insulator 35.

In this embodiment, a tube of a transparent resin film was used as the material of the cylinder member 35a composing the insulator 35.

Thus, according to the syringe for tympanic injection 30 according to the present embodiment, in spite of the tendency for the reversible temperature indicators 20, 25 provided on the outer surface 12b of the barrel 12 to indicate a temperature lower than the temperature of the real infusion under the influence of the outside air, the reversible temperature indicators 20, 25 can indicate a temperature close to the temperature of the infusion due to the reduction of the effect of the outside air by the insulator 35. Accordingly, it becomes possible to recognize the temperature of the infusion more precisely and to recognize more precisely that the infusion in the barrel 12 is at a temperature within the injection temperature range suitable for the administration into the tympanic cavity.

In addition, the infusion in the barrel 12 is kept warm by the insulator 35 covering the substantially whole area of the outer surface 12b of the barrel 12, making it possible to maintain within a preferable temperature range for longer time the temperature of the infusion in the syringe for intratympanic administration 30 removed from the state where the syringe is warmed.

Moreover, since the visual recognition of the color change of the reversible temperature indicators 20, 25 through the insulator 35 is possible, the user recognizes the presence or absence of the color change of the reversible temperature indicators 20, 25 with the insulator 35 attached to allow the injection into the tympanic cavity. Thus, it is not necessary to remove the insulator 35 from the outer surface 12b of the barrel 12 before the injection of the infusion into the tympanic cavity to confirm the presence or absence of the color change of the reversible temperature indicators 20, 25.

Furthermore, the barrel 12 is kept warm by the air layer composing the sealed space layer C between the insulator 35 and the outer surface 12b of the barrel 12 and the decrease of temperature of the infusion in the barrel 12 is further suppressed.

Moreover, by setting the distance between the inner face of the cylinder member 35a and the outer surface of the syringe at 0.5 mm or more and 2.0 mm or less, the infusion in the barrel 12 can be kept warm more effectively.

In the present embodiment, a substantially whole area of the outer surface 12b of the barrel 12 is covered with the insulator 35 and the syringe has the sealed space layer C, but a variety of the modifications are possible without limiting thereto.

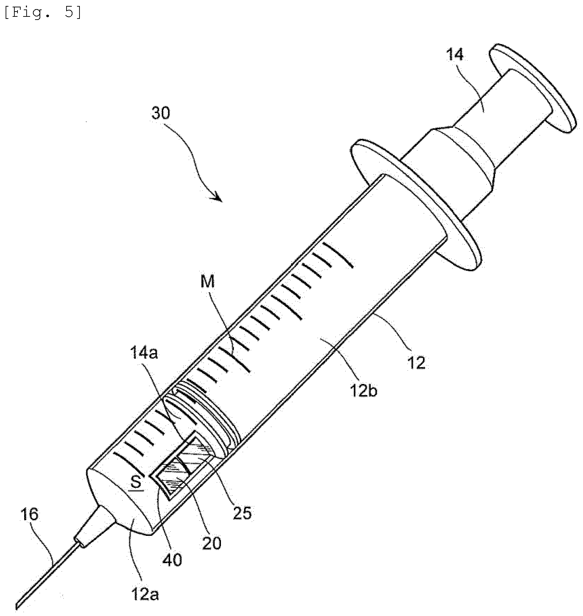

The first modification of the insulator is described below with reference to Fig. 5. In Fig. 5, the same elements as those in the embodiment described in Figs. 1-4 described above are indicated with the same reference signs and the descriptions thereof are omitted. Fig. 5 is a perspective view illustrating the syringe for tympanic injection 30 according to the modification of the insulator.

In this modification, a substantially rectangle sheet insulator 40, instead of the insulator 35 composed of the cylinder member 35a and the like, is affixed onto the outer surface 12b of the barrel 12 such that the insulator covers the first reversible temperature indicator 20 and the second reversible temperature indicator 25, as illustrated in the figure.

The insulator 40 is in close contact with each of the outer surface 12b of the barrel, the first reversible temperature indicator 20, and the second reversible temperature indicator 25, and no air layer is provided.

According to the syringe for tympanic injection 30 having the insulator 40 according to this modification, in spite of the tendency for the reversible temperature indicators 20, 25 provided on the outer surface 12b of the barrel 12 to indicate a temperature lower than the temperature of the real infusion under the influence of the outside air, the reversible temperature indicators 20, 25 can indicate a temperature close to the temperature of the infusion due to the reduction of the effect of the outside air by the insulator 40. Accordingly, it becomes possible to recognize the temperature of the infusion more precisely and to recognize more precisely that the infusion in the barrel 12 is at a temperature within the injection temperature range suitable for the administration into the tympanic cavity.

The present invention is not limited to the aforementioned embodiment, but a variety of modifications are possible as long as they do not deviate from the scope of the claims. For example, while the first reversible temperature indicator 20 and the second reversible temperature indicator 25 are used as temperature indicators in the aforementioned embodiment, the third reversible temperature indicator 45 (additional reversible temperature indicator, see Table 3) that changes in color at 37°C may be provided on the outer surface 12b of the barrel 12 in the injection temperature range (34°C to 39°C) defined beforehand by the first reversible temperature indicator 20 and the second reversible temperature indicator 25.

According to this, since the third reversible temperature indicator 45 (further reversible temperature indicator) changes in color at 37°C in the injection temperature range (34°C to 39°C) suitable for the administration into the tympanic cavity defined by the first reversible temperature indicator 20 and the second reversible temperature indicator 25 as illustrated in Table 3, it can be recognized whether the infusion is at a temperature in the higher temperature region or the lower temperature region with a border thereof at 37°C in the injection temperature range suitable for the administration into the tympanic cavity.

Therefore, the user of the syringe can recognize the tendency of the temperature change of the infusion within the injection temperature range suitable for the administration into the tympanic cavity and avoid the situation where the temperature of the infusion becomes out of the temperature range for the injection in a short period of time after the confirmation of the reversible temperature indicators and before the injection of the infusion into a patient because the user is unaware that the infusion is at a temperature in the lower temperature region.

### [Example 1]

The present invention will be described with reference to Examples below.

### 1-1. Production of syringe

As the reversible temperature indicators affixed to the outer surface of the barrels of the syringes illustrated in Table 4 below, "Thermo Pit Liquid Crystal Sticker" (product number: N-26-46D, a product made by AS ONE Corporation) was used. 11 liquid crystal temperature indicators in total are printed on a piece of black tape as numbers at 2°C intervals from 26°C to 46°C. The liquid crystal temperature indicators develop a bright green color when the color development temperature is reached and exhibit a light blue color or a light orange color at -1°C and +1°C of the color development temperature. Therefore, for example, at 36°C, a liquid crystal temperature indicator of 36°C develops a bright green color and at 37°C 2 adjacent liquid crystal temperature indicators of 36°C and 38°C exhibit a light blue color or a light orange color simultaneously, and therefore the temperature can be determined to be 37°C. The color of the liquid crystal temperature indicators disappears and becomes the same black as the tape when the temperature is out of the color development temperature. In this Example, 30 mm ^{∗} 6 mm stickers cut at both ends in the longitudinal direction were prepared leaving a display part from 30°C to 40°C of "Thermo Pit Liquid Crystal Sticker". They are hereinafter referred to as "Thermo Pit Liquid Crystal Stickers 30-40". The "Thermo Pit Liquid Crystal Sticker" used may be considered as an assemble of the first to sixth reversible temperature indicators that exhibit a bright green color at 30°C, 32°C, 34°C, 36°C, 38°C, and 40°C, respectively.

In the following Examples, when only the liquid crystal temperature indicator of 30°C exhibited a light orange color upon temperature decrease of the infusion, it was read as 29°C.

"Thermo Pit Liquid Crystal Stickers 30-40" were affixed such that the 30°C side was placed at the tip of the barrel and the 40°C side was placed at the flange of the barrel.

The tip of the barrel is closed with a cap, Japanese Pharmacopoeia "water for injection" was placed into the barrel from the rear of the barrel according to the volume of the syringe, and then the rear of the barrel was sealed with a waterproof tape (waterproof flashing tape 8067, a product made by 3M Japan Limited).

**[Table 4]**

| Syringe No. | No. 1 | No. 2 | No. 3 | No. 4 | No. 5 | No. 6 |
|---|---|---|---|---|---|---|
| Manufacturer /Seller | Taisei Kako Co., Ltd | Daikyo Seiko, Ltd. | Terumo Corporation | Tsubasa Industry Co., Ltd. | | |
| Volume | 1 ml | 1 ml | 2.5 ml | 1 ml | 2 ml | 5 ml |
| Material | Cyclic polyolefin | Made of Resin CZ | Polypropylene | Heat-resistant glass | Heat-resistant glass | Heat-resistant glass |
| Outer diameter of syringe | 9.2 mm | 7.8 mm | 9.9 mm | 9.9 mm | 12.3 mm | 18.4 mm |
| Inner diameter of syringe | 6.2 mm | 4.6 mm | 8.3 mm | 6.5 mm | 9.4 mm | 15.0 mm |
| Wall thickness of syringe | 1.5 mm | 1.6 mm | 0.8 mm | 1.7 mm | 1.5 mm | 1.7 mm |
| Length of syringe | 65 mm | 63 mm | 60 mm | 65 mm | 85 mm | 85 mm |
| Material of plunger | Rigid polyethylene | Rigid polyethylene | Polypropylene | Heat-resistant glass | Heat-resistant glass | Heat-resistant glass |
| Length of plunger | 60 mm | 58 mm | 65 mm | 66 mm | 80 mm | 86 mm |

### 1-2. Measurement of temperature change of infusion in syringe (barrel)

Syringes No. 1 to 6 filled with "water for injection" are placed on their sides for 30 minutes in an incubator set at 40°C to warm the syringes and the infusion. Subsequently, the syringes are taken out and a sensor of a needle probe thermometer (digital core thermometer, product number: AD-5625, a product made by A&D Company, Limited) was inserted at the center of a waterproof tape on the rear of the barrel to measure the temperature of the infusion. The thermo-sensor at the tip of the probe was fixed to the center of the infusion and the syringe was placed on its side and the measurement was started immediately. The environment of the measurement room was adjusted at 20±1°C unless stated specifically.

The determination of the indicated temperature of the needle probe thermometer and "Thermo Pit Liquid Crystal Sticker 30-40" was made at 15 second intervals from the start of measurement to 60 seconds after the start and then at 30 second intervals. Since the display of "Thermo Pit Liquid Crystal Sticker 30-40" is at 2°C intervals, the temperature when adjacent liquid crystal temperature indicators exhibit a light color simultaneously was read as the intermediate temperature between them. For example, the temperature when the adjacent liquid crystals of 34°C and 36°C exhibit a light color simultaneously is determined to be 35°C. The time point at which the temperature of the infusion becomes 29°C or less was determined to be the measurement end point.

Table 5 illustrates the results of the determination of the measurement temperature with the needle probe thermometer and the displayed temperature of "Thermo Pit Liquid Crystal Sticker 30-40" over time. In the table, Liquid temperature indicates the liquid temperature of the "water for injection" measured with the needle probe thermometer, Liquid crystal indicates the temperature of "Thermo Pit Liquid Crystal Sticker 30-40", and Difference indicates the temperature difference of the both. Difference is expressed as an integer value that is rounded off.

Moreover, the mean, the maximum, and the minimum values of Difference with syringe Nos. 1 to 6 are illustrated in Table 6.

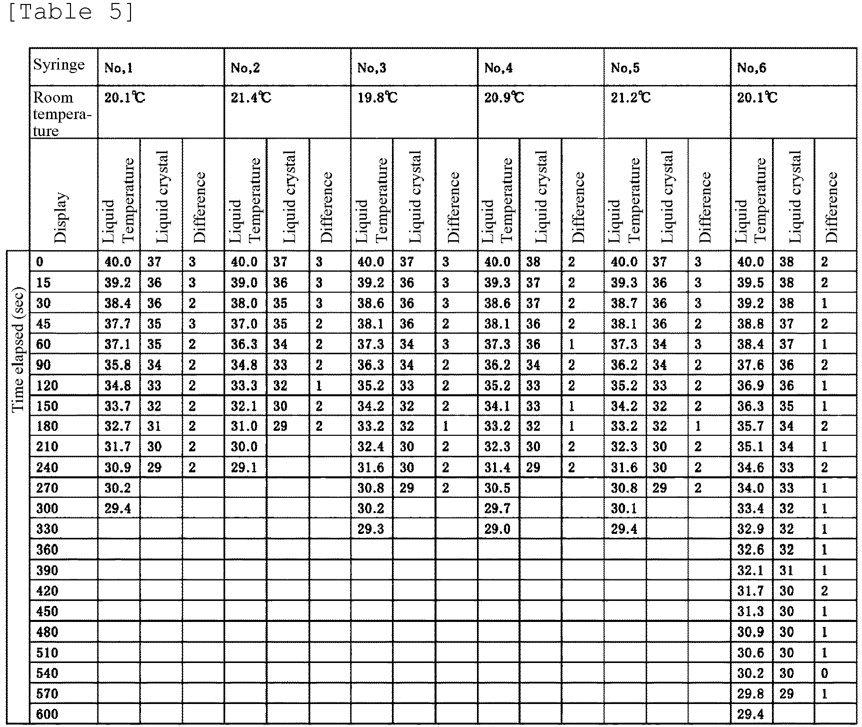

**[Table 6]**

| Syringe No. | No. 1 | No. 2 | No. 3 | No. 4 | No. 5 | No. 6 |
|---|---|---|---|---|---|---|
| Mean | 2 | 2 | 2 | 2 | 2 | 1 |
| Maximum | 3 | 3 | 3 | 2 | 3 | 2 |
| Minimum | 2 | 1 | 1 | 1 | 1 | 0 |

From Tables 5 and 6, it was found that although there is a difference between the temperature of the infusion and the temperature of the liquid crystal display, the decrease of temperature of the contained liquid is followed by that of the liquid crystal display. Moreover, according to Table 6, the mean of difference between the temperature of the infusion and the liquid crystal display in syringes No. 1 to No. 5 was 2°C and the mean of difference between the temperature of the infusion and the liquid crystal display in syringe No. 6 was 1°C.

Therefore, it was found that the difference should be considered to confirm the temperature of the infusion with the liquid crystal display. The reason why the difference in syringe No. 6 is small is considered to be that the temperature decrease in syringe No. 6 is slower than other syringes since syringe No. 6 has a larger volume of an infusion than those of the other syringes and the temperature of the reversible temperature indicators is easy to follow the temperature change of the infusion.

Moreover, while the syringe of No. 6 took approximately 5 minutes until the temperature of the infusion became below 34°C after the warming to 40°C, syringes No. 1 to No. 5 took 1 minute 30 seconds to 3 minutes to become below 34°C.

Thus, it was confirmed that while no countermeasure to the decrease of temperature is necessary for syringes with a large volume of infusion such as that of No. 6 since the temperature of the infusion in such syringes decreases slowly and the importance of making visual recognition of the temperature of the infusion possible and the importance of countermeasures to suppress the decrease of temperature are high since the temperature of the infusion in syringes with small volumes used for the intratympanic administration of an infusion decreases rapidly.

By Examples below, the temperature of the infusion and the display of the reversible temperature indicator and the difference thereof were determined without an insulator and with a variety of insulators. Since the present invention is for the intratympanic administration, the syringe numbers No. 1 (a volume of 1 ml), No. 2 (a volume of 1 ml), and No. 4 (a volume of 1 ml) were selected for the syringes to be examined.

### [Example 2]

Tests to confirm the effect of providing an insulator on the outer surface of the barrel of syringe No. 1 on heat retention of the infusion and on the difference between the temperature of the infusion and the displayed temperature of the liquid crystal were conducted.

### 2-1. Production of syringe with insulator

The same 5 syringes as syringe No. 1 produced in [Example 1] were produced and covered around the whole outer surface of the barrel and over the length of the barrel on the outer surface of the barrel with 5 sheet insulators of No. 1 to No. 5 set forth in Table 7 below to produce syringe No. 1-1 to No. 1-5, respectively.

For syringes No. 1-1 to No. 1-4, an insulator was in close contact with the outer surface of the barrel. For syringe No. 1-5, foam backing double-sided tapes (a product number: 516L, a product made by Sekisui Chemical Co., Ltd.) with a width of 1 mm × a thickness of 1.3 mm were wound in rings (which refers to 35b in Figs. 3 and 4 and is also referred to as the spacers) around the both ends of the barrel in the longitudinal direction and a sealed space layer was provided between the outer surface of the barrel and the insulator by forming the cylinder member by placing the insulator No. 2 set forth in Table 7 over the insulators around the barrel.

**[Table 7]**

| Insulator No. | No. 1 | No. 2 | No. 3 | No. 4 | No. 5 * |
|---|---|---|---|---|---|
| Product name/number | Minafoam(R) closed-cell foam sheet 105 | Transparent insulation film DN-T01 | Insulation sheet Clear E1450 | Nanoballoon(R) CA | Transparent insulation film DN-T01 (cylinder member), foam substrate Minafoam product number 115 (spacer) |
| Manufacturer /Seller | Sakai Chemical Industry Co., Ltd. | Kikuchi Fusuma Manufacturing Co., Ltd | Nitoms, Inc. | Toyohozai Co. Ltd. | Kikuchi Fusuma Manufacturing Co.,Ltd Sekisui Chemical Co.,Ltd. |
| Thickness of insulator | 0.5 mm | 0.10 mm | 1.25 mm | 0.01 mm | 1.5-mm sealed air layer |
| Material | Foamed polyethylene | Polyester | Special polyethylene | Multilayer polyester film having silica hollow particle layer as intermediate layer | Polyester, Polyethylene foam |
| Properties | Semitransparent white allowing distinguishing of color change of reversible temperature indicator | Colorless transparent | Colorless transparent Having air layer with open ends | Colorless transparent | Colorless transparent |

### 2-2. Measurement of temperature change of infusion in syringe (barrel)

By the same method as in "1-2. Measurement of temperature change of infusion in syringe (barrel)" in [Example 1], the temperatures of the infusion in syringes No. 1-1 and No. 1-5 were measured and the display of "Thermo Pit Liquid Crystal Stickers 30-40" was confirmed.

The results are illustrated in Table 8. Moreover, the mean, the maximum, and the minimum of the differences in syringes No. 1 and No. 1-1 to No. 1-5 are illustrated in Table 9 below.

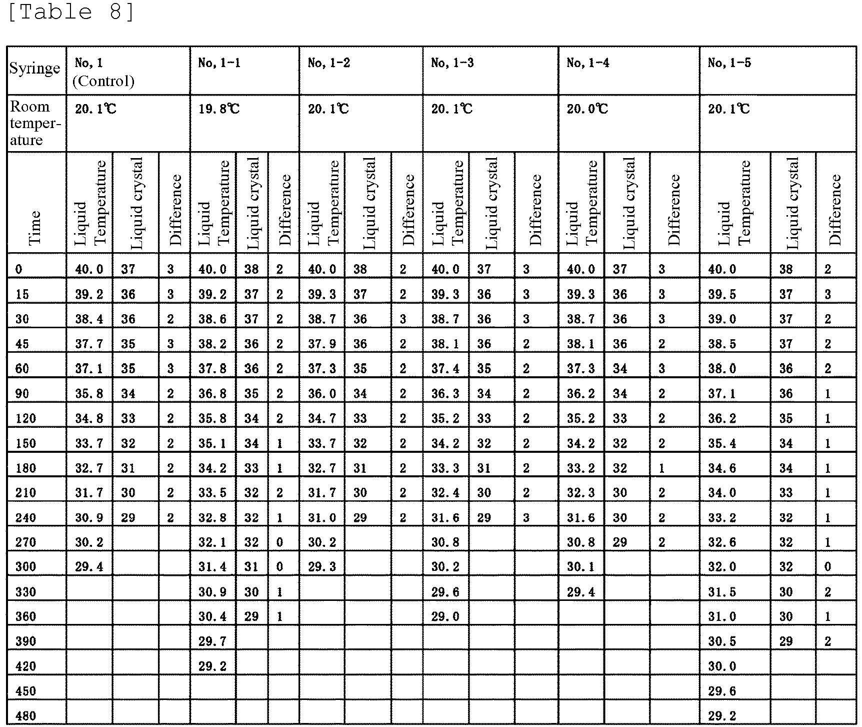

**[Table 9]**

| Syringe No. | No. 1 | No. 1-1 | No. 1-2 | No. 1-3 | No. 1-4 | No. 1-5 |
|---|---|---|---|---|---|---|
| Mean | 2 | 1 | 2 | 3 | 2 | 1 |
| Maximum | 3 | 2 | 3 | 3 | 3 | 2 |
| Minimum | 2 | 0 | 2 | 2 | 1 | 0 |

According to Tables 8 and 9, the result that the difference between the temperature of the infusion and the displayed temperature of the reversible temperature indicators is small in the syringes with the insulators of No. 1-1 and No. 1-5 in comparison with the syringe without the insulator No. 1 was obtained. This is considered to be because syringes No. 1-1 and No. 1-5 have a high thermal-insulation effect and therefore the reversible temperature indicators are hard to be affected by the outside air, the speed of temperature decrease of the infusion became low, and the temperature around the reversible temperature indicators became easy to follow the temperature of the infusion. Furthermore, the effect that inhibits the decrease of the temperature of the infusion was confirmed in the both syringes.

### [Example 3]

Tests to confirm the effect of providing an insulator on the outer surface of the barrel of syringe No. 2 on heat retention of the infusion and on the difference between the temperature of the infusion and the displayed temperature of the liquid crystal were conducted.

### 3-1. Production of syringe with insulator

The same 5 syringes as syringe No. 2 produced in [Example 1] were produced and covered around the whole outer surface of the barrel and over the length of the barrel on the outer surface of each barrel with 5 sheet insulators of No. 1 to No. 5 set forth in Table 7 above to produce syringe No. 2-1 to No. 2-5.

For syringes No. 2-1 to No. 2-4, the insulator and the outer surface of the barrel are in close contact. For syringe No. 2-5, the space between the insulator and the outer surface of the barrel is a sealed space layer.

### 3-2. Measurement of temperature change of infusion in syringe (barrel)

By the same method as in "1-1. Measurement of temperature change of infusion in syringe (barrel)" in [Example 1], the temperatures of the infusion in syringes No. 2-1 to No. 2-5 were measured and the display of "Thermo Pit Liquid Crystal Stickers 30-40" was confirmed.

The results are illustrated in Tables 10 and 11. Table 10 illustrates the mean, the maximum, and the minimum of the differences in syringes No. 2 and No. 2-1 to No. 2-5.

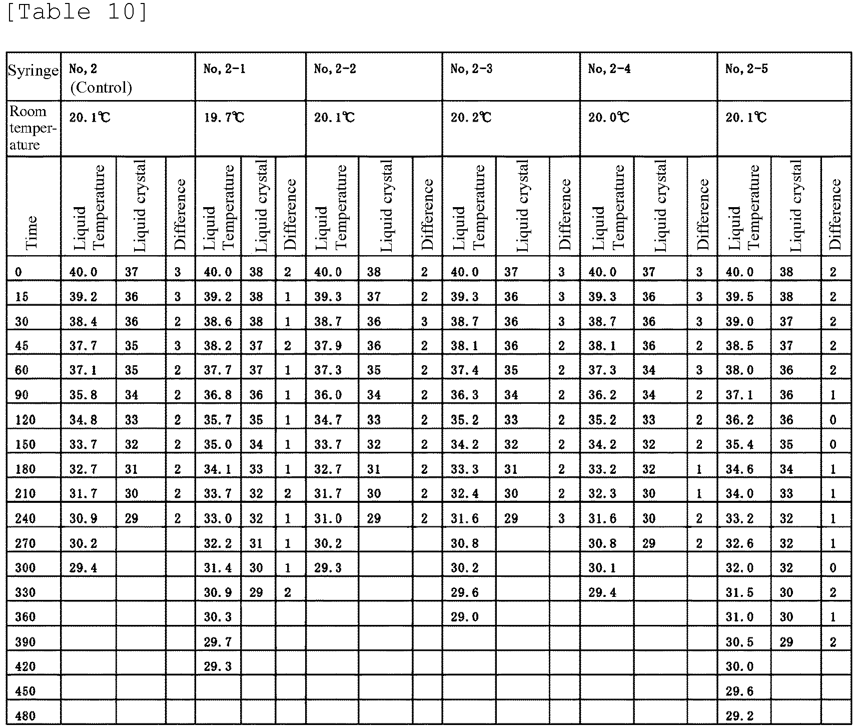

**[Table 11]**

| Syringe No. | No. 2 | No. 2-1 | No. 2-2 | No. 2-3 | No. 2-4 | No. 2-5 |
|---|---|---|---|---|---|---|
| Mean | 2 | 1 | 2 | 3 | 2 | 2 |
| Maximum | 3 | 2 | 3 | 3 | 3 | 2 |
| Minimum | 1 | 1 | 1 | 2 | 2 | 1 |

According to Tables 10 and 11, the result that the difference between the temperature of the infusion and the displayed temperature of the reversible temperature indicators is small in the syringes with the insulators of No. 2-1 and No. 2-5 in comparison with the syringe without the insulator No. 2 was obtained. This is considered to be because syringes No. 2-1 and No. 2-5 have a high thermal-insulation effect and therefore the reversible temperature indicators are hard to be affected by the outside air, the speed of temperature decrease of the infusion became low, and the temperature around the reversible temperature indicators became easy to follow the temperature of the infusion. Furthermore, the effect that inhibits the decrease of the temperature of the infusion was confirmed in the both syringes.

### [Example 4]

Tests to confirm the effect of providing an insulator on the outer surface of the barrel of No. 4 syringe on heat retention of the infusion and on the difference between the temperature of the infusion and the displayed temperature of the liquid crystal were conducted.

### 4-1. Production of syringe with insulator

The same 5 syringes as syringe No. 4 produced in [Example 1] were produced and covered around the whole outer surface of the barrel and over the length of the barrel on the outer surface of each barrel with 5 sheet insulators of No. 1 to No. 5 set forth in Table 7 above to produce syringe No. 4-1 to No. 4-5.

For syringes No. 4-1 to No. 4-4, the insulator and the outer surface of the barrel are in close contact. For syringe No. 4-5, the space between the insulator and the outer surface of the barrel is a sealed space layer.

### 4-2. Measurement of temperature change of infusion in syringe (barrel)

By the same method as in "1-1. Measurement of temperature change of infusion in syringe (barrel)" in [Example 1], the temperatures of the infusion in syringes No. 4-1 to No. 4-5 were measured and the display of "Thermo Pit Liquid Crystal Stickers 30-40" was confirmed.

The results are illustrated in Tables 12 and 13. Table 13 illustrates the mean, the maximum, and the minimum of the differences in syringes No. 4 and No. 4-1 to No. 4-5.

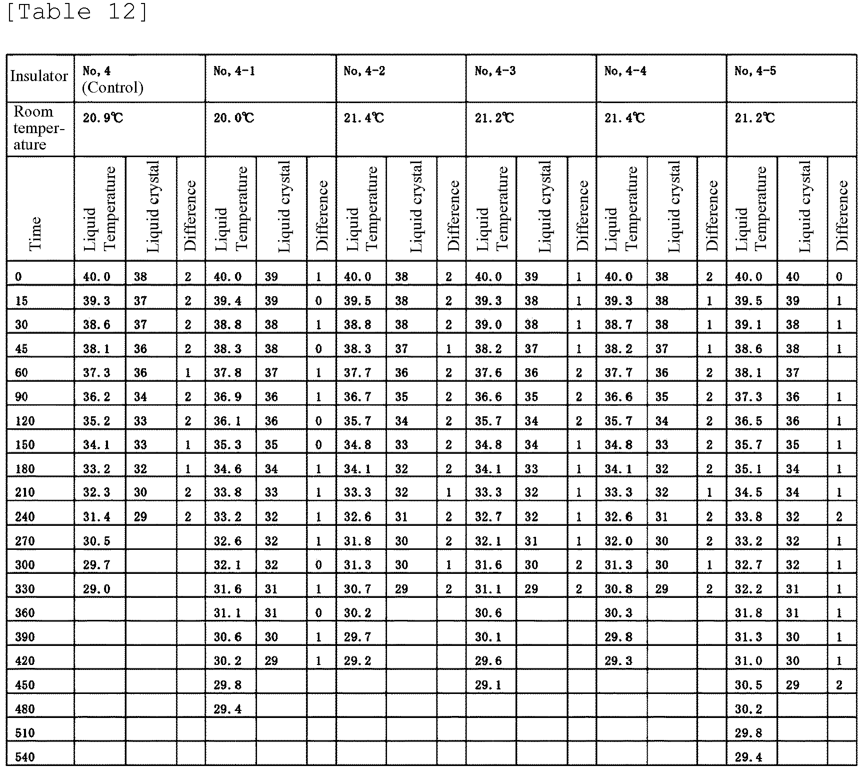

**[Table 13]**

| Syringe No. | No. 4 | No. 4-1 | No. 4-2 | No. 4-3 | No. 4-4 | No. 4-5 |
|---|---|---|---|---|---|---|
| Mean | 2 | 1 | 2 | 1 | 2 | 1 |
| Maximum | 2 | 1 | 2 | 2 | 2 | 2 |
| Minimum | 1 | 0 | 0 | 1 | 1 | 0 |

According to Tables 12 and 13, the result that the difference between the temperature of the infusion and the displayed temperature of the reversible temperature indicators is small in the syringes with the insulators of No. 4-1, No. 4-3 and No. 4-5 in comparison with the syringe without the insulator No. 4 was obtained. This is considered to be because syringes No. 4-1, No. 4-3, and No. 4-5 have a high thermal-insulation effect and therefore the reversible temperature indicators are hard to be affected by the outside air, the speed of temperature decrease of the infusion became low, and the temperature around the reversible temperature indicators became easy to follow the temperature of the infusion. Moreover, the effect that inhibits the decrease of the temperature of the infusion was confirmed in syringes No. 4-2 to No. 4-5 and the effect was markedly strong in syringes No. 4-1 and No. 4-5.

### [Example 5]

5 closed-cell foam insulator sheets different in thickness were provided on the outer surface of the barrel of syringe No. 1 and the relations between the thickness of the sheets and the heat retention of the infusion and the difference between the temperature of the infusion and the displayed temperature of the liquid crystal were tested. The distinction of the color change of the reversible temperature indicators was confirmed in combination.

### 5-1. Production of syringe with insulator

Each one of the 2 temperature indicators, 10 mm × 5 mm of the first reversible temperature indicator (made by Japan Capsular Products Inc., special specification: exhibiting a black color at 38°C or less, and being colorlessness at 39°C or more) and 10 mm × 5 mm of the second reversible temperature indicator (made by Japan Capsular Products Inc., special specification: exhibiting a red color in 33°C or less and being colorlessness at 34°C or more), was affixed side by side to the tip (the side close to the needle) on the outer surface of the barrel of syringe No. 1 in the longitudinal direction of this barrel. At this point of time, the colors of the first and second reversible temperature indicators were black and red, respectively. The same 5 syringes as this syringe were produced and a whole area of the outer surface of the barrel on the outer surface of each barrel was covered with a closed-cell foam insulator sheet "Minafoam(R)" with a length of 65 mm, which is the length of the barrel. The syringe covered with the insulator sheet with a thickness of 0.15 mm (special specifications) was referred to as No. 1-6, the syringe covered with the insulator sheet with a thickness of 0.3 mm (special specifications) was referred to as No. 1-7, the syringe covered with the insulator sheet with a thickness of 0.5 mm (product number #105) was referred to as No. 1-8, the syringe covered with the insulator sheet with a thickness of 1 mm (product number #110) was referred to as No. 1-9, and the syringe covered with a thickness of 1.5 mm (special specifications) was referred to as No. 1-10.

### 5-2. Measurement of temperature change of infusion in syringe (barrel)

By the same method as in "1-1. Measurement of temperature change of infusion in syringe (barrel)" in [Example 1], the temperature change of the infusion was measured. The colors of 2 temperature indicators were confirmed through an insulation sheet in combination.

The results are illustrated in Table 14.

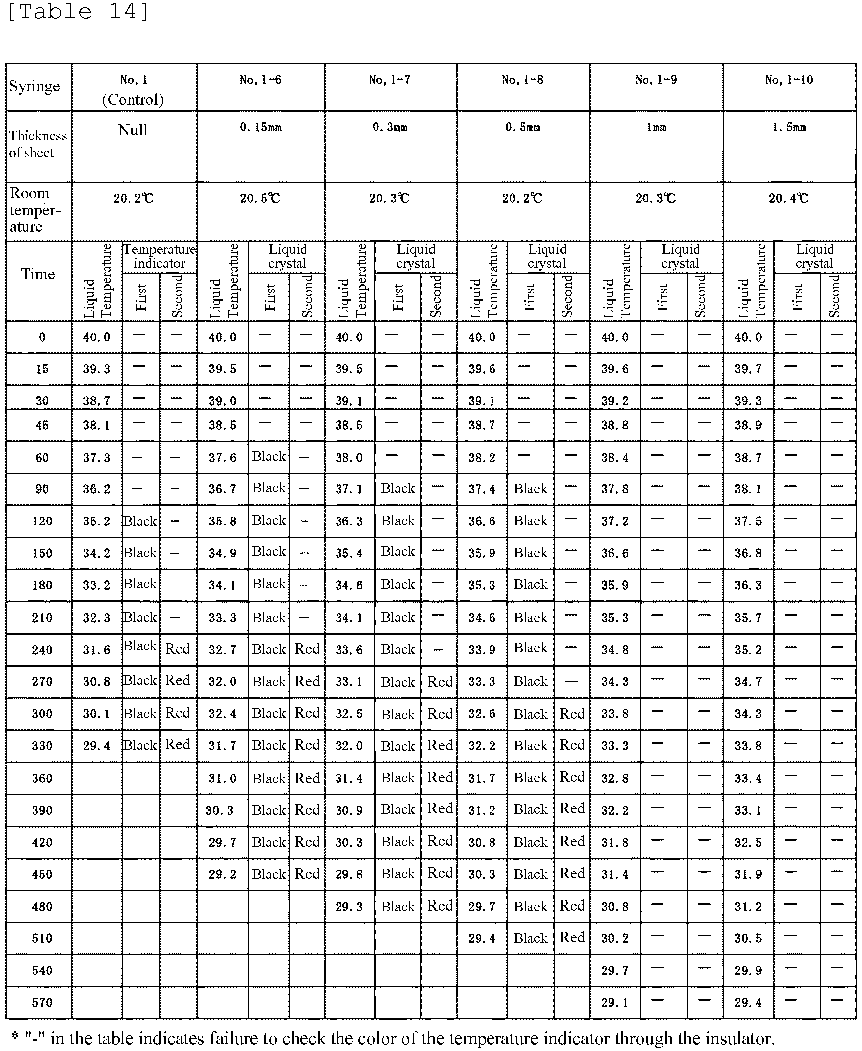

In syringes No. 1-6, No. 1-7, and No. 1-8 having a closed-cell foam insulator sheet with a thickness of 0.15 mm, 0.3 mm, or 0.5 mm, the colors of 2 reversible temperature indicators could be confirmed through the insulation sheet. Moreover, while the color change of 2 reversible temperature indicators was approximately 60 seconds delayed from the temperature of the infusion in syringe No. 1 with no insulator, but there was almost no delay in syringes No. 1-6, No. 1-7, and No. 1-8. In syringes No. 1-9 and No. 1-10 having an insulator sheet with a thickness of 1 mm or 1.5 mm, there was no transparency and the color could not be confirmed.

### [Example 6]

In the syringes having a sealed space layer between the outer surface of the barrel and the inner face of the insulator, tests to confirm the effect of the distance between the inner face of the insulator and the outer surface of the barrel (that is, the thickness of the sealed space layer around the barrel) on the insulation effect were conducted.

### 6-1. Production of syringe having sealed space layer

The same 6 syringes as syringe No. 1 produced in [Example 1] were produced and foam substrates (Minafoam product number #110) each with a width of 1 mm and a different thickness were wound around the both ends of the barrel of each syringe in the longitudinal direction to provide ring-shaped spacers 35b made of the insulator. The transparent insulation film DN-T01 of No. 2 in Table 7 above was placed over these ring-shaped insulators around the barrel to form a cylinder member and a sealed space layer with air was provided between the outer surface of the barrel and the insulator (see Figs. 3 and 4).

The thickness h of the sealed space layer of each syringe is adjusted to the thickness of the spacers 35b. A syringe No. 1-A (having a sealed space layer with a thickness of 0.3 mm) having spacers 35b made by cutting Minafoam with special specifications (with a thickness of 0.3 mm) in a width of 1 mm and winding the pieces around the both ends of the barrel, a syringe No. 1-B (having a sealed space layer with a thickness of 0.5 mm) having spacers 35b made by cutting Minafoam product number #105 (with a thickness of 0.5 mm) in a width of 1 mm and winding the pieces around the both ends of the barrel, a syringe No. 1-C (having a sealed space layer with a thickness of 1 mm) having spacers 35b made by cutting Minafoam product number #110 (with a thickness of 1 mm) in a width of 1 mm and winding the pieces around the both ends of the barrel, a syringe No. 1-D (having a sealed space layer with a thickness of 1.5 mm) having spacers 35b made by cutting Minafoam with special specifications (with a thickness of 1.5 mm) in a width of 1 mm and winding the pieces around the both ends of the barrel, a syringe No. 1-E (having a sealed space layer with a thickness of 2 mm) having spacers 35b made by cutting Minafoam product number #120 (with a thickness of 2 mm) in a width of 1 mm and winding the pieces around the both ends of the barrel, a syringe No. 1-F (having a sealed space layer with a thickness of 2.5 mm) having spacers 35b made by cutting Minafoam with special specifications (with a thickness of 2.5 mm) in a width of 1 mm and winding the pieces around the both ends of the barrel, and a syringe No. 1-G (having a sealed space layer with a thickness of 3 mm) having spacers 35b made by cutting Minafoam product number #130 (with a thickness of 3 mm) in a width of 1 mm and winding the pieces around the both ends of the barrel were produced.

### 6-2. Measurement of temperature change of infusion in syringe (barrel)

The temperature change of the infusion in syringes No. 1 and No. 1-A to No. 1-G was measured by the same method as in "1-1. Measurement of temperature change of infusion in syringe (barrel)" in [Example 1].

The results are illustrated in Tables 15 and 16. Table 16 illustrates the mean, the maximum, and the minimum of differences in syringes No. 1 and No. 1-A to No. 1-G.

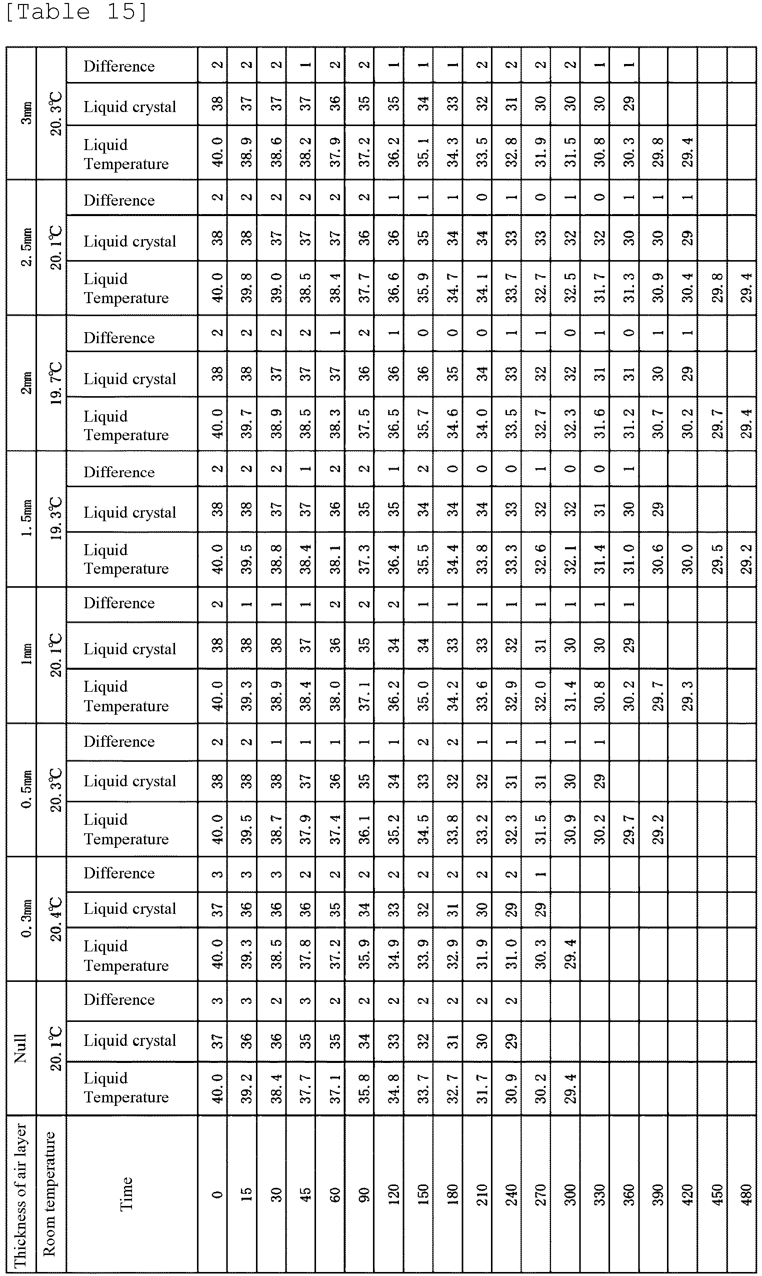

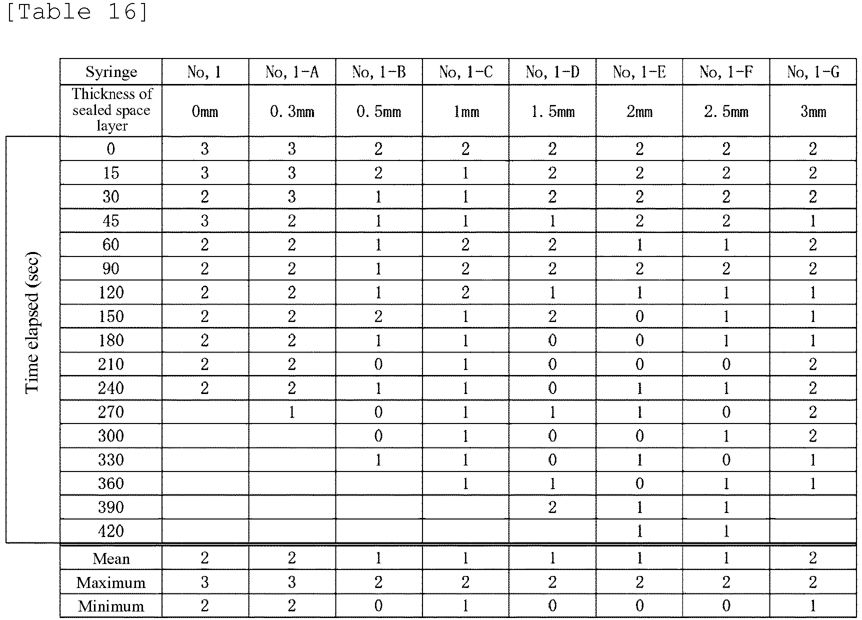

According to Tables 15 and 16, the difference is smaller in syringe No. 1-B having a sealed space layer with a thickness of 0.5 mm and syringe No. 1-F having a sealed space layer with a thickness of 2 mm in comparison with syringe No. 1 and the temperature of the infusion and the display of the reversible temperature indicators were almost same. Moreover, the decrease of the temperature of the infusion was suppressed. In syringe No. 1-A having a sealed space layer with a thickness of 0.3 mm, there was no effect on the difference and the decrease of the temperature. Moreover, in syringes No. 1-F and No. 1-G having a sealed space layer with a thickness of 2.5 mm or 3 mm, the insulation effect was the same or decreased in comparison with syringe No. 1-E having a sealed space layer with a thickness of 2 mm.

### [Example 7]

When only the surfaces of the reversible temperature indicators were covered with an insulator, the effect on the difference between the temperature of the infusion and the display of the reversible temperature indicators was examined.

### 7-1. Production of syringe to be used in tests

One of "Thermo Pit Liquid Crystal Stickers 30-40" was affixed to the outer surface of the barrel of syringe No. 1 such that the longitudinal direction of this "Thermo Pit Liquid Crystal Sticker 30-40" extends in the longitudinal direction of the barrel and another one of "Thermo Pit Liquid Crystal Stickers 30-40" was affixed to a site on the outer surface that is on the other side of the already affixed "Thermo Pit Liquid Crystal Sticker 30-40". And one of the 2 pieces in total of "Thermo Pit Liquid Crystal Stickers 30-40" was covered with the insulator No. 5 in Table 7 above having a fit size. The other piece of "Thermo Pit Liquid Crystal Stickers 30-40" was not covered with any insulator and exposed to outside air. This syringe is hereinafter referred to as No. 1-5p.

7-2. Measurement of temperature change of infusion in syringe (barrel)

The temperature change of the infusion in syringe No. 1-5p was measured by the same method as in "1-1. Measurement of temperature change of infusion in syringe (barrel)" in [Example 1]. The display of 2 pieces of liquid crystal stickers was confirmed in combination.

The results are illustrated in Tables 17 and 18. Table 17 illustrates the mean, the maximum, and the minimum of the difference in syringe No. 1-5p.

**[Table 17]**

| Room temperature | 19.9 | | | | |
|---|---|---|---|---|---|
| Time | Liquid Temperature | Liquid crystal (without insulation) | | Liquid crystal (with insulation) | |
| | | Display | Difference | Display | Difference |
| 0 | 40.0 | 39 | 1 | 40 | 0 |
| 15 | 39.1 | 38 | 1 | 39 | 0 |
| 30 | 38.6 | 37 | 2 | 38 | 1 |
| 45 | 38.2 | 36 | 2 | 38 | 0 |
| 60 | 37.7 | 35 | 3 | 37 | 1 |
| 90 | 36.4 | 35 | 1 | 36 | 0 |
| 120 | 35.5 | 34 | 2 | 35 | 1 |
| 150 | 34.5 | 33 | 2 | 34 | 1 |
| 180 | 33.6 | 32 | 2 | 33 | 1 |
| 210 | 32.7 | 31 | 2 | 33 | 0 |
| 240 | 32.1 | 29 | 3 | 32 | 0 |
| 270 | 31.3 | 29 | 2 | 30 | 1 |
| 300 | 30.7 | 29 | 2 | 30 | 1 |
| 330 | 30.1 | | | 29 | 1 |
| 360 | 29.6 | | | | |
| 390 | 29.0 | | | | |

**[Table 18]**

| Syringe | No. 1-5P | |
|---|---|---|
| With or without insulation | Liquid crystal (without insulation) | Liquid crystal (with insulation) |
| Mean | 2 | 1 |
| Maximum | 3 | 1 |
| Minimum | 1 | 0 |

According to Tables 17 and 18, it was found that the difference between the temperature of the infusion and the displayed temperature of the liquid crystal decreases just by covering only the reversible temperature indicator with an insulator.

### [Example 8]

The effect of the difference of the temperature of the environment (room temperature) in which the syringe is used after warming on the difference between the temperature of the infusion and the displayed temperature of the reversible temperature indicators was examined. In this Example, syringe No. 1-5 was used.

### 8-1. Measurement of temperature change of infusion in syringe (barrel)

By the same method as in "1-1. Measurement of temperature change of infusion in syringe (barrel)" in [Example 1], the temperature of the infusion in syringe No. 1-5 was measured and the display of "Thermo Pit Liquid Crystal Stickers 30-40" was examined. The measurement was made at room temperatures of 15°C and 25°C and the results were compared with the result of [Example 2] with syringe No. 1-5, which was measured at a room temperature of 20°C.

The results are illustrated in Tables 19 and 20. Table 20 illustrates the mean, the maximum, and the minimum of the difference in syringe No. 1-5.

**[Table 19]**

| Syringe | No, 1-5 | | | | | | | | |
|---|---|---|---|---|---|---|---|---|---|
| Room temperature | 15.1°C | | | 20.1°C | | | 25.3°C | | |
| Time | Liquid Temperature | Liquid crystal (insulation) | Difference | Liquid Temperature | Liquid crystal (insulation) | Difference | Liquid Temperature | Liquid crystal (insulation) | Difference |
| 0 | 40.0 | 38 | 2 | 40.0 | 38 | 2 | 40.0 | 40 | 0 |
| 15 | 38.7 | 38 | 1 | 39.5 | 37 | 2 | 39.6 | 40 | 0 |
| 30 | 38.2 | 37 | 1 | 39.0 | 37 | 2 | 39.4 | 40 | 1 |
| 45 | 37.5 | 37 | 1 | 38.5 | 37 | 2 | 39.1 | 39 | 0 |
| 60 | 37.2 | 35 | 2 | 38.0 | 36 | 2 | 38.5 | 38 | 1 |
| 90 | 36.3 | 35 | 1 | 37.1 | 36 | 1 | 37.4 | 37 | 0 |
| 120 | 35.4 | 34 | 1 | 36.2 | 35 | 1 | 36.3 | 36 | 0 |
| 150 | 33.8 | 33 | 1 | 35.4 | 34 | 1 | 35.3 | 36 | 1 |
| 180 | 32.7 | 32 | 1 | 34.6 | 34 | 1 | 34.7 | 35 | 0 |
| 210 | 31.8 | 31 | 1 | 34.0 | 33 | 1 | 34.8 | 35 | 0 |
| 240 | 30.9 | 30 | 1 | 33.2 | 32 | 0 | 34.3 | 34 | 0 |
| 270 | 30.0 | 29 | 1 | 32.6 | 32 | 1 | 33.8 | 34 | 0 |
| 300 | 29.4 | | | 32.0 | 32 | 0 | 33.5 | 33 | 1 |
| 330 | | | | 31.5 | 30 | 2 | 33.1 | 33 | 0 |
| 360 | | | | 31.0 | 30 | 1 | 32.9 | 33 | 0 |
| 390 | | | | 30.5 | 29 | 2 | 32.3 | 33 | 1 |
| 420 | | | | 30.0 | | | 32.1 | 32 | 0 |
| 450 | | | | 29.6 | | | 31.9 | 32 | 0 |
| 480 | | | | 29.2 | | | 31.6 | 32 | 0 |
| 510 | | | | 29.3 | | | 31.4 | 31 | 0 |
| 540 | | | | | | | 31.1 | 31 | 0 |
| 570 | | | | | | | 30.9 | 31 | 0 |
| 600 | | | | | | | 30.7 | 31 | 0 |
| 630 | | | | | | | 30.5 | 30 | 1 |
| 660 | | | | | | | 30.3 | 30 | 0 |
| 690 | | | | | | | 30.2 | 30 | 0 |
| 720 | | | | | | | 30.0 | 30 | 0 |
| 750 | | | | | | | 29.9 | 30 | 0 |
| 780 | | | | | | | 29.8 | 30 | 0 |
| 810 | | | | | | | 29.6 | 29 | 1 |
| 840 | | | | | | | 29.5 | | |
| 870 | | | | | | | 29.3 | | |

**[Table 20]**

| Syringe | No. 1-5 | | |
|---|---|---|---|
| Mean | 1 | 1 | 0 |
| Maximum | 2 | 2 | 1 |
| Minimum | 1 | 0 | 0 |

According to Tables 19 and 20, the differences between the temperature of the infusion and the displayed temperature of the liquid crystals were equal to or less than 2°C in the room temperatures of 15°C and 20°C. Moreover, the decrease of the temperature of the infusion was extremely slow at the room temperature 25°C. Therefore, it was found that a countermeasure to the decrease of the temperature of the infusion in the barrel with a small volume is necessary when the room temperature is a temperature below 25°C.

### [Example 9]

A syringe made of cyclic polyolefin (a volume of 1 ml) from Taisei Kako Co., Ltd. was filled sterilely with 0.6 ml of an aqueous solution of 4.3 mg/ml dexamethasone sodium phosphate (4 mg/ml in terms of dexamethasone phosphate) and sealed with a cap and a plunger. Both ends of an assembly of the reversible temperature indicators of "Thermo Pit Liquid Crystal Stickers" N-26-46D commercially available from AS ONE Corporation. was cut to leave 3 reversible temperature indicators for 34°C, 36°C, and 38°C. It is an assembly tape of 25 mm × 5 mm on which the 3 reversible temperature indicators that exhibit a bright green when it reaches each temperature, a dark blue or orange at the temperature ± 1°C, and black of the groundwork at the other temperatures were printed as numbers and was affixed to the outer surface of the barrel such that it extends in the longitudinal direction of the barrel.

A foam backing double-sided tape (made by Sekisui Chemical Co., Ltd., product number 516L, special polyethylene foam, acryl adhesive) with a width of 1 mm and a thickness of 1.3 mm was wound around the both ends of the barrel as ring-shaped insulators to form spacers. The barrel was covered with a polyester film cylinder member of a thickness of 0.1 mm over these spacers. A 1.3 mm sealed space layer was provided around the barrel in this way.

The syringe produced as described above was placed in the personal incubator JP culture III (made by J.P Clarus Co., Ltd.) and warmed for 20 minutes. The syringe was taken out from the incubator and the assembly of the reversible temperature indicators was confirmed to exhibit the number 36 in a bright green.

As a result of the administration of the infusion at such a temperature into the tympanic cavity in 10 patients with sudden hearing loss, no patients appealed for dizziness or nausea due to the administration,

### [Example 10]

A syringe made of CZ resin (with a volume of 1 ml and an outer diameter of barrel of 7.8 mm) from Daikyo Seiko, Ltd. was filled sterilely with 0.6 ml of an aqueous solution of 5 mg/ml amoxicillin, an antimicrobial agent and sealed with a cap and a plunger. The first reversible temperature indicator ("Thermowappen(R)" made by NiGK Corporation, special specification: exhibiting a bright green color at 38°C ± 1°C and a black color of the groundwork within the other temperature ranges, 5 mm × 5 mm square) and the second reversible temperature indicator ("Thermowappen(R)" made by NiGK Corporation, special specification: exhibiting a bright green color at 35°C ± 1°C and a black color of the groundwork within the other temperature ranges, 5 mm × 5 mm square) were affixed side by side to the tip (the side near the needle tip) on the outer surface of the barrel such that there was no overlap. Furthermore, the surface thereof was covered with "Nanoballoon film CA" of No. 4 in Table 7 as an insulator. At this point of time, 2 Thermowappens(R) were black. The syringe was placed in the personal incubator JP culture III (made by J.P Clarus Co., Ltd.) and warmed for 30 minutes. The syringe was taken out from the incubator and the 2 Thermowappens(R) were examined to confirm that only the Thermowappen(R) for 38°C exhibited a bright green color. When they were examined 3 minutes later, only the Thermowappen(R) for 36°C exhibited a bright green color.

As a result of the administration of the agent in the injector in the state where only the Thermowappen(R) for 36°C exhibited a bright green color into the tympanic cavity in 7 patients with sudden hearing loss, no patients appealed for dizziness or nausea due to the administration.

### [Example 11]

A syringe made of cyclic polyolefin CZ resin (with a volume of 1 ml and an outer diameter of barrel of 9.2 mm) from Taisei Kako Co., Ltd. was filled sterilely with 1.0 ml of an aqueous solution of 2% gentamicin and sealed with a cap and a plunger. The first reversible temperature indicator ("Temperature-sensitive dye sticker" from Japan Capsular Products Inc., special specification: exhibiting a black color at 37°C or less and changing into colorless and transparent at 38°C or more, belt-shaped with a width of 3 mm) and the second reversible temperature indicator ("Temperature-sensitive dye sticker" made by Japan Capsular Products Inc., special specification: exhibiting a red color at 33°C or less and changing into colorlessness and transparent at 34°C or more, belt-shaped; with a width of 3 mm) were wound around the tip (the side near the needle tip) on the outer surface of the barrel in rings (in the circumferential direction of the outer surface of the barrel) and 2 pieces were affixed adjacent to each other in the longitudinal direction of the barrel.

Furthermore, the barrel was covered with a closed-cell foam insulation sheet ("Minafoam" with special specifications made by Sakai Chemical Industry Co., Ltd.) with a thickness of 0.3 mm on the surface thereof. The syringe was placed in the program incubator IN800 (made by Yamato Scientific Co., Ltd. and set at 39°C) and warmed for 15 minutes. The syringe was taken out from the incubator and the temperature-sensitive dye stickers were examined and it was confirmed that the first and second stickers of the 2 temperature-sensitive dye stickers were both white. When the temperature-sensitive dye stickers were examined 5 minutes later, the first temperature-sensitive dye sticker was black and the second temperature-sensitive dye sticker was white.

As a result of the administration of the agent in the syringe in the state where the first temperature-sensitive dye sticker is black and the second temperature-sensitive dye sticker is white into the tympanic cavity in 6 patients with sudden hearing loss, no patients appealed for dizziness or nausea due to the administration,

### Reference Signs List

10, 30 Syringe for tympanic injection
12 Barrel
12b Outer surface
20, 50 First reversible temperature indicator
25, 52 Second reversible temperature indicator
35a (35) Cylinder member (insulator)
35b (35) ring-shaped insulator (insulator)
40 Insulator
45 Third reversible temperature indicator (additional reversible temperature indicator)

## Claims

1. A syringe (10, 30) for tympanic injection for directly injecting an infusion in a barrel (12) into a tympanic cavity, comprising
a plurality of reversible temperature indicators (20, 25, 45, 50, 52) provided on an outer surface of the barrel (12), the plurality of reversible temperature (20, 25, 45, 50, 52) indicators each reversibly changing in color at a different temperature, **characterized in that**:
the plurality of reversible temperature indicators (20, 25, 45, 50, 52) comprising a lower limit reversible temperature indicator (20, 50) that changes in color at a lower limit of a range of injection temperature suitable for administration into the tympanic cavity and an upper limit reversible temperature indicator (25, 52) that changes in color at an upper limit of the range of injection temperature suitable for administration into the tympanic cavity; **in that**
a color change of the lower limit reversible temperature indicator (20, 50) and the upper limit reversible temperature indicator (25, 52) allows visual recognition of a range of injection temperature suitable for administration into the tympanic cavity; and **in that**
the reversible temperature indicators (20, 25, 45, 50, 52) comprise at least three reversible temperature indicators provided and
the at least three reversible temperature indicators (20, 25, 45, 50, 52) comprise the lower limit reversible temperature indicator (20, 50) and the upper limit reversible temperature indicators (25, 52) defining the range of injection temperature suitable for administration into the tympanic cavity and an additional reversible temperature (45) indicator changing in color in the range of injection temperature suitable for administration into the tympanic cavity.

2. The syringe (10, 30) for tympanic injection according to claim 1, wherein the lower limit of the range of injection temperature suitable for administration into the tympanic cavity is 34 °C and the upper limit of the range of injection temperature suitable for administration into the tympanic cavity is 39 °C.

3. The syringe (10, 30) for tympanic injection according to any one of claims 1 or 2, wherein an insulator (35, 40) covering the reversible temperature indicators (20, 25, 45, 50, 52) is provided on an outer surface of the barrel.

4. The syringe (10, 30) for tympanic injection according to claim 3, wherein the insulator (35, 40) is provided to cover a substantially whole area of the outer surface of the barrel (12).

5. The syringe (10, 30) for tympanic injection according to claim 3 or 4, wherein the insulator (35, 40) has a degree of transparency allowing visual recognition of color of the reversible temperature indicators (20, 25, 45, 50, 52) covered by the insulator (35, 40).

6. The syringe (10, 30) for tympanic injection according to any one of claims 3 to 5, wherein a space between the insulator (35, 40) and the outer surface (12b) of the barrel (12) is an enclosed space layer.

7. The syringe (10, 30) for tympanic injection according to claim 6, wherein a distance between an inner face of the insulator (35, 40) and the outer surface (12b) of the barrel (12) is 0.5 mm or more and 2.0 mm or less.

## Patentansprüche

1. Spritze (10, 30) zur Mittelohrinjektion zum direkten Injizieren einer Infusion in einem Zylinder (12) in eine Paukenhöhle, wobei die Spritze Folgendes umfasst:
mehrere umkehrbare Temperaturanzeiger (20, 25, 45, 50, 52), die an einer Außenfläche des Zylinders (12) bereitgestellt werden, wobei die mehreren umkehrbaren Temperaturanzeiger (20, 25, 45, 50, 52) jeweils bei einer unterschiedlichen Temperatur umkehrbar die Farbe ändern, **dadurch gekennzeichnet, dass**:
die mehreren umkehrbaren Temperaturanzeiger (20, 25, 45, 50, 52) einen umkehrbaren Untergrenzen-Temperaturanzeiger (20, 50), der an einer unteren Grenze eines Injektionstemperaturbereichs, der zum Verabreichen in die Paukenhöhle geeignet ist, die Farbe ändert, und einen umkehrbaren Obergrenzen-Temperaturanzeiger (25, 52), der an einer oberen Grenze des Injektionstemperaturbereichs, der zum Verabreichen in die Paukenhöhle geeignet ist, die Farbe ändert, umfassen, dadurch, dass
eine Farbänderung des umkehrbaren Untergrenzen-Temperaturanzeigers (20, 50) und des umkehrbaren Obergrenzen-Temperaturanzeigers (25, 52) ein visuelles Erkennen eines Injektionstemperaturbereichs, der zum Verabreichen in die Paukenhöhle geeignet ist, ermöglicht, und dadurch, dass
die umkehrbaren Temperaturanzeiger (20, 25, 45, 50, 52) mindestens drei bereitgestellte umkehrbare Temperaturanzeiger umfassen und
die mindestens drei umkehrbaren Temperaturanzeiger (20, 25, 45, 50, 52) den umkehrbaren Untergrenzen-Temperaturanzeiger (20, 50) und den umkehrbaren Obergrenzen-Temperaturanzeiger (25, 52) umfassen, die den Injektionstemperaturbereich definieren, der zum Verabreichen in die Paukenhöhle geeignet ist, und einen zusätzlichen umkehrbaren Temperaturanzeiger (45), der in dem Injektionstemperaturbereich, der zum Verabreichen in die Paukenhöhle geeignet ist, die Farbe ändert.

2. Spritze (10, 30) zur Mittelohrinjektion nach Anspruch 1, wobei die untere Grenze des Injektionstemperaturbereichs, der zum Verabreichen in die Paukenhöhle geeignet ist, 34 °C beträgt und die obere Grenze des Injektionstemperaturbereichs, der zum Verabreichen in die Paukenhöhle geeignet ist, 39 °C beträgt.

3. Spritze (10, 30) zur Mittelohrinjektion nach einem der Ansprüche 1 oder 2, wobei ein Isolator (35, 40), der die umkehrbaren Temperaturanzeiger (20, 25, 45, 50, 52) abdeckt, an einer Außenfläche des Zylinders bereitgestellt wird.

4. Spritze (10, 30) zur Mittelohrinjektion nach Anspruch 3, wobei der Isolator (35, 40) bereitgestellt wird, um im Wesentlichen ein gesamtes Gebiet der Außenfläche des Zylinders (12) abzudecken.

5. Spritze (10, 30) zur Mittelohrinjektion nach Anspruch 3 oder 4, wobei der Isolator (35, 40) einen Grad an Transparenz aufweist, der ein visuelles Erkennen einer Farbe der umkehrbaren Temperaturanzeiger (20, 25, 45, 50, 52), die durch den Isolator (35, 40) abgedeckt werden, ermöglicht.

6. Spritze (10, 30) zur Mittelohrinjektion nach einem der Ansprüche 3 bis 5, wobei ein Raum zwischen dem Isolator (35, 40) und der Außenfläche (12b) des Zylinders (12) eine Schicht eines eingeschlossenen Raums ist.

7. Spritze (10, 30) zur Mittelohrinjektion nach Anspruch 6, wobei ein Abstand zwischen einer Innenseite des Isolators (35, 40) und der Außenfläche (12b) des Zylinders (12) 0,5 mm oder mehr und 2,0 mm oder weniger beträgt.

## Revendications

1. Seringue (10, 30) pour injection tympanique, pour injecter directement une perfusion dans un cylindre (12) dans une cavité du tympan, comprenant
une pluralité d'indicateurs de température réversibles (20, 25, 45, 50, 52) agencés sur une surface externe du cylindre (12), la pluralité d'indicateurs de température réversibles (20, 25, 45, 50, 52) changeant chacun de couleur de manière réversible à une température différente, **caractérisée en ce que** :
la pluralité d'indicateurs de température réversibles (20, 25, 45, 50, 52) comprend un indicateur de température réversible de limite inférieure (20, 50) qui change de couleur à une limite inférieure d'une plage de températures d'injection appropriées pour une administration dans la cavité du tympan, et un indicateur de température réversible de limite supérieure (25, 52) changeant de couleur à une limite supérieure de la plage de températures d'injection appropriées pour une administration dans la cavité du tympan ; **en ce que**
un changement de couleur de l'indicateur de température réversible de limite inférieure (20, 50) et de l'indicateur de température réversible de limite supérieure (25, 52) permet une reconnaissance visuelle d'une plage de températures d'injection appropriées pour une administration dans la cavité du tympan ; et **en ce que** :
les indicateurs de température réversibles (20, 25, 45, 50, 52) comprennent au moins trois indicateurs de température réversibles fournis et,
les au moins trois indicateurs de température réversibles (20, 25, 45, 50, 52) comprennent l'indicateur de température réversible de limite inférieure (20, 50) et l'indicateur de température de limite supérieure (25, 52) définissant la plage de températures d'injection appropriées pour une administration dans la cavité du tympan, et un indicateur de température réversible additionnel (45) changeant de couleur dans la plage de températures d'injection appropriées pour une injection dans la cavité du tympan.

2. Seringue (10, 30) pour injection tympanique selon la revendication 1, dans laquelle la limite inférieure de la plage de températures d'injection appropriées pour une administration dans la cavité du tympan correspond à 34°C et la limite supérieure de la plage de températures d'injection appropriées pour une injection dans la cavité du tympan correspond à 39°C.

3. Seringue (10, 30) pour injection tympanique selon l'une quelconque des revendications 1 ou 2, dans laquelle un isolant (35, 40) recouvrant les indicateurs de température réversibles (20, 25, 45, 50, 52) est fourni sur une surface externe du cylindre.

4. Seringue (10, 30) pour injection tympanique selon la revendication 3, dans laquelle l'isolant (35, 40) est fourni pour recouvrir une zone sensiblement complète de la surface externe du cylindre (12).

5. Seringue (10, 30) pour injection tympanique selon la revendication 3 ou 4, dans laquelle l'isolant (35, 40) a un degré de transparence permettant une reconnaissance visuelle de la couleur des indicateurs de température réversibles (20, 25, 45, 50, 52) recouverts par l'isolant (35, 40).

6. Seringue (10, 30) pour injection tympanique selon l'une quelconque des revendications 3 à 5, dans laquelle un espace entre l'isolant (35, 40) et la surface externe (12b) du cylindre (12) est une couche d'espace renfermé.

7. Seringue (10, 30) pour injection tympanique selon la revendication 6, dans laquelle une distance entre une face interne de l'isolant (35, 40) et la surface externe (12b) du cylindre (12) correspond à 0,5 mm ou plus et à 2,0 mm ou moins.
